# EUROPÄISCHE PATENTSCHRIFT

(11) **EP 3 649 998 B1**
(45) Veröffentlichungstag und Bekanntmachung des Hinweises auf die Patenterteilung: **22.12.2021**
(21) Anmeldenummer: 19205653.9
(22) Anmeldetag: 28.10.2019
(51) Int. Cl.: A61F 13/534, A61F 13/537, A61F 13/53

(54) **EINWEG-ABSORPTIONSARTIKEL, INSBESONDERE EINWEG-WINDEL ODER EINWEG-WINDELHÖSCHEN, MIT LEICHTEM, HOCHSAUGFÄHIGEM ABSORPTIONSKERN**
DISPOSABLE ABSORBENT PRODUCT, IN PARTICULAR DISPOSABLE NAPPY OR DISPOSABLE NAPPY PANTS, WITH A LIGHT, HIGHLY ABSORBENT CORE
ARTICLE D'ABSORPTION JETABLE, EN PARTICULIER COUCHE JETABLE OU COUCHE-CULOTTE JETABLE, POURVU DE NOYAU D'ABSORPTION LÉGER ET FORTEMENT ABSORBANT

(30) Priorität: 07.11.2018 DE 102018127771
(43) Veröffentlichungstag der Anmeldung: 13.05.2020
(73) Patentinhaber: L&R Vertriebs GmbH, 71720 Oberstenfeld (DE)
(72) Erfinder: Mayer, Ralf, 71720 Oberstenfeld (DE); Kravtsov, Maria, 1009 Pully (CH)
(74) Vertreter: Rüger Abel Patentanwälte PartGmbB

(56) Entgegenhaltungen:
- WO-A1-2007/139154
- DE-U1- 9 317 553
- JP-B2- 6 234 492
- US-A- 5 599 336

## Beschreibung

Die vorliegende Erfindung betrifft einen Einweg-Absorptionsartikel und insbesondere eine Einweg-Windel oder ein Einweg-Windelhöschen mit einem leichten, hochsaugfähigem Absorptionskern.

Einweg-Absorptionsartikel, wie etwa Einweg-Windeln oder Einweg-Windelhöschen für Babys, Übungshöschen für Kleinkinder und Inkontinenzunterwäsche für Erwachsene, dienen dazu, Urin, Kot und andere ausgeschiedene Körperflüssigkeiten eines Trägers schnell zu absorbieren und sicher zurückzuhalten, um ein Austreten der Körperausscheidungen nach außen und eine damit verbundene Benässung oder Verunreinigung von Kleidung, Bettwäsche oder anderen mit dem Absorptionsartikel in Kontakt tretenden Gegenständen zu verhindern. Es müssen zum Teil beträchtliche Mengen einer Ausscheidungsflüssigkeit, wie Urin, innerhalb kurzer Zeit von der Innenfläche der Windel weg und in Absorptionsschichten der Windel hinein abgeleitet werden, um einen Rückstau auf der Innenfläche zu verhindern. Die Windel sollte in der Lage sein, die Flüssigkeit, z.B. für eine Nachtruhe von Babys, für 12 Stunden oder mehr halten zu können und auch unter Druck nicht wieder zurückzugeben. Ein derartiger unter Druck verursachte Rückfluss von Flüssigkeit wird auf dem vorliegenden Fachgebiet auch als Reflux bezeichnet. Trotz eines zuverlässigen Auslaufschutzes sollte eine Windel auch atmungsaktiv sein, um einen Hitzestau in der Windel zu vermeiden. Im Übrigen sind insbesondere für Babywindeln besonders weiche, antiallergische, hautverträgliche Materialien, die toxisch unbedenklich und umweltverträglich sind, eine hohe Passform und einen hohen Tragekomfort der Windel ermöglichen, erwünscht. Zu weiteren gewünschten Eigenschaften für Windeln gehören eine geringe Dicke und hohe Flexibilität, damit sie leicht und angenehm angewendet und möglichst kompakt verpackt, transportiert und gelagert werden können. Sämtliche hier vor- und nachstehend in Bezug auf Windeln gemachten Ausführungen gelten, sofern aus dem Kontext nichts anderes hervorgeht, in gleicher Weise auch für Windelhöschen, einschließlich Übungshöschen und Inkontinenzunterwäsche, und andere ähnliche Einweg-Absorptionsprodukte, ohne dass diese explizit erwähnt werden.

Um die vorstehenden Funktionen zu erfüllen und die gewünschten Eigenschaften zu erreichen, sind Absorptionsartikel, wie Windeln, meist aus mehreren Schichten aufgebaut und weisen einen absorbierenden Kern auf, der in einem Grundkörper aufgenommen ist. Im Allgemeinen weist der Grundkörper eine obere Lage auf, die im Einsatz mit der Haut des Trägers unmittelbar in Kontakt steht und aus einem weichen, hydrophilen Material, bspw. Vlies, Baumwolle oder dgl., geschaffen sein kann. Eine untere Lage, die auf der körperfernen Seite der Windel angeordnet ist, umfasst meist eine flüssigkeitsundurchlässige Folie, bspw. aus Polypropylen, Polyethylen oder dgl., als Auslaufschutz sowie eine Außenschlicht aus einem weichen Textilmaterial, bspw. Vlies, Baumwolle oder dgl. Zwischen der oberen und der unteren Lage ist der Absorptionskern angeordnet, der ein Absorptionsmaterial zur Absorption und Speicherung der Ausscheidungsflüssigkeit aufweist. Zwischen der oberen Lage und dem Absorptionskern kann optional eine Aufnahme- und Verteilungsschicht eingefügt sein, um die Flüssigkeit schnell von der oberen Lage zu dem Absorptionskern abzuleiten und sie möglichst breit in der Fläche zu verteilen, um das Absorptionsmaterial in dem Absorptionskern wirksam zu nutzen.

Das Absorptionsmaterial des Absorptionskerns ist speziell dazu eingerichtet, eine Flüssigkeit schnell zu absorbieren und für eine längere Zeitdauer zurückzuhalten, einen Reflux zu vermeiden und so die Haut des Trägers trocken zu halten. Herkömmliche Absorptionskerne von Absorptionsartikeln sind gewöhnlich aus saugfähigen holzhaltigen Fasern, insbesondere Zellulosefasern (auch als Pulp bezeichnet) zusammengesetzt, wobei in dem absorbierenden Kern oft zusätzlich ein absorbierendes Material verteilt eingebettet ist, um die Absorptionskapazität zu erhöhen. Das absorbierende Material kann ein superabsorbierendes Polymer (SAP) sein, das ein vernetztes Polymermaterial ist, das in der Lage ist, ein Mehrfaches, bspw. wenigstens das Zehnfache seines Körpergewichts an Flüssigkeit (Frischwasser) zu speichern. Bei der Aufnahme der Flüssigkeit quillt das SAP-Material auf und bildet ein Hydrogel. Das SAP-Material wird auch als "polymeres Absorptionsmaterial", "gelierendes Absorptionsmaterial" oder einfach als "Superabsorber" bezeichnet. Es besteht der Wunsch, Absorptionsartikel mit einem Absorptionskern zu schaffen, die eine hohe Absorptionsgeschwindigkeit und hohe Absorptionskapazität bei geringem Gewicht und geringem Reflux aufweisen.

EP 3 205 318 A1 beschreibt einen Absorptionskern für einen Einweg-Absorptionsartikel mit wenigstens einer ersten und einer zweiten Absorptionsschicht, die jeweils ein faserförmiges Absorptionsmaterial aufweisen, das in Längsrichtung des Kerns gleichmäßig angeordnet ist. Die Absorptionsschichten sind kontinuierlich und übereinanderliegend angeordnet. Das Absorptionsmaterial der ersten Absorptionsschicht weist ein superabsorbierendes Material auf, während das Material der zweiten Absorptionsschicht im Wesentlichen frei von einem superabsorbierenden Material ist. Das Absorptionsvermögen der zweiten Absorptionsschicht ohne superabsorbierendes Material kann begrenzt sein.

WO 2014/151544 A2 beschreibt einen Absorptionskern aus einem synthetischen Faserbündel- oder Vliesmaterial, das eine Kernmatrix bildet, in oder an der zwei unterschiedliche SAP-Materialien oder -Gemische angeordnet sind, die sich hinsichtlich ihres Absorptionsverhaltens unterscheiden. Ein erstes SAP ist langsam wirkend, weist jedoch eine hohe Absorptionskapazität auf, während ein anderes SAP schnell wirkend ist, jedoch ein geringes Absorptionsvermögen aufweist. Die Kernmatrix mit den SAP-Materialien ist in eine Hülle aus einem nicht absorbierenden thermoplastischen Fasergeflecht oder Vlies eingehüllt, die verhindert, dass das SAP aus der Kernfasermatrix herausfällt. Für den Kernaufbau ist eine Vielzahl unterschiedlicher Materialien erforderlich, und der Fertigungsaufwand kann hoch sein.

DE 11 2008 000 010 B4 beschreibt einen Einweg-Absorptionsartikel, z.B. eine Windel, mit einem Absorptionskern, der zellulosefrei ausgeführt ist. Der Absorptionskern weist eine erste und eine zweite Absorptionsschicht auf, die teilchenförmiges, polymeres Superabsorbermaterial enthalten, das auf einem ersten bzw. zweiten Substrat angeordnet ist. Die teilchenförmigen, polymeren Absorptionsmaterialien sind auf den jeweiligen Substraten jeweils in diskreten Mustern angeordnet bzw. diskontinuierlich verteilt und derart miteinander kombiniert, dass die jeweiligen Muster aus teilchenförmigem polymerem Absorptionsmaterial gegeneinander versetzte Inselbereiche definieren, die über Verbindungsbereiche aus thermoplastischem Haftmaterial miteinander verbunden und immobilisiert sind. Das thermoplastische Haftmaterial kann das Gewicht und die Dicke des Absorptionsartikels erhöhen. Außerdem sind thermoplastische Haftmaterialien, die frei von toxisch und ökologisch bedenklichen Bestandteilen sind, kostspielig.

DE 698 33 127 T2 schlägt vor, zur Erhöhung der Flüssigkeitsaufnahmefähigkeiten des absorbierenden Kerns zwei oder mehrere absorbierende Schichtbereiche vorzusehen, die ein superabsorbierendes Polymermaterial aufweisen und in spezieller Weise miteinander wechselwirken, um die Absorptionsfähigkeiten zu erhöhen. Die superabsorbierenden Polymermaterialien sind in Fasern in zwei oder mehreren Schichten eingemischt. Die speziellen Superabsorber sind mit einer gewünschten gesteuerten Rate ausgewählt, die auch mit der Partikelgröße konfiguriert sein kann, um ein bestimmtes Oberflächenbereich-zu-VolumenVerhältnis zu erhalten. Die Partikelgrößen des superabsorbierenden Materials sollten in allen absorbierenden Schichtbereichen nicht weniger als 140 Mikrometer und nicht mehr als 1000 Mikrometer betragen.

EP 3 357 466 A1 beschreibt einen Absorptionsartikel mit einem Absorptionskern, der zwei polymere Absorptionsschichten aufweist, die ein superabsorbierendes Polymer (SAP) enthalten und zwischen denen eine hydrophile Stoffschicht angeordnet ist. Der Absorptionskern ist von einer Hüllsandwichstruktur umgeben, die zwei hydrophile Faserschichten und eine dazwischen eingefügte Zelluloseschicht aufweist. Zellulosefasern der Zelluloseschicht dringen durch die darunterliegende hydrophile Faserschichte und in den Absorptionskern hinein und stehen mit dem SAP-Material in Kontakt, um eine schnelle Ableitung der Flüssigkeit zu fördern.

DE 93 17 553 U1 beschreibt einen Saugkörper für Windeln oder Inkontinentenvorlagen mit einem Saugkissen und einem granulierten superabsorbierenden Material (Superabsorber), wobei auf der körpernahen Seite des Saugkissens eine Grobkornkomponente des Superabsorbers und auf der körperfernen Seite des Saugkissens eine Feinkornkomponente des Superabsorbers angeordnet sind. Das Saugkissen kann aus gekräuselter Polyesterwatte, einem Vliesstoff oder einem anderen saugaktiven Material bestehen, das hydrophil ist, und dient dazu, ankommende Flüssigkeit, die die Grobkomponente des Superabsorbers passiert, aufzunehmen und zu der Feinkornkomponente des Superabsorbers passieren zu lassen. Eine Hüllschicht in Form einer flüssigkeitsundurchlässigen Folie umschließt die körperferne Seite und umgreift Ränder der körpernahen Seite des Saugkissens, wobei die Feinkornkomponente des Superabsorbers auf der Innenseite der Hüllschicht mittels einer Leimschicht fixiert ist. Eine Deckschicht deckt die körpernahe Seite der Anordnung des Saugkörpers ab, und die Grobkomponente des Superabsorbers ist mittels einer Leimschicht an der Innenseite der Deckschicht fixiert.

Es besteht weiterhin ein Bedarf, die Leistungsfähigkeit von Einweg-Absorptionsartikeln, wie Windeln oder Windelhöschen, hinsichtlich der Absorptionsgeschwindigkeit und -kapazität bei reduziertem Gewicht und einfachem Aufbau des Absorptionskerns unter Verwendung unbedenklicher Kernmaterialien zu verbessern.

Ausgehend hiervon ist es eine Aufgabe der Erfindung einen Einweg-Absorptionsartikel zu schaffen, der bei einem geringen Gewicht und relativ einfachem Aufbau des Absorptionskerns eine gute Flüssigkeitsabsorption mit hoher Absorptionsgeschwindigkeit und hoher Absorptionskapazität, eine schnelle Trocknungszeit von Oberflächen, eine gute Verteilung der Flüssigkeit in dem Absorptionskern und einen geringen Reflux ermöglicht.

Zur Lösung dieser Aufgabe ist ein Einweg-Absorptionsartikel geschaffen, der die Merkmale des unabhängigen Anspruchs 1 aufweist. Besonders bevorzugte Ausführungsformen der Erfindung sind Gegenstand von Unteransprüchen.

Der Einweg-Absorptionsartikel gemäß der Erfindung weist einen Grundkörper und einen Absorptionskern auf. Der Grundkörper weist eine im Wesentlichen flüssigkeitsdurchlässige obere Lage, die im Einsatz mit der Körperhaut eines Trägers in Kontakt gelangt, und eine untere Lage auf einer gegenüberliegenden, körperfernen Seite auf. Der Absorptionskern ist zwischen der oberen Lage und der unteren Lage des Grundkörpers angeordnet und weist eine erste Absorptionsschicht und eine zweite Absorptionsschicht zur Absorption einer durch den Einweg-Absorptionsartikel aufgenommenen Flüssigkeit auf. Die erste und die zweite Absorptionsschicht sind in der Dickenrichtung zwischen der oberen und der unteren Lage übereinanderliegend angeordnet. Die erste Absorptionsschicht weist ein teilchenförmiges superabsorbierendes Polymermaterial mit einem ersten Teilchendurchmesser auf, und die zweite Absorptionsschicht weist ein teilchenförmiges superabsorbierendes Polymermaterial mit einem zweiten Teilchendurchmesser auf. Der zweite Teilchendurchmesser ist kleiner als der erste Teilchendurchmesser. Zwischen der ersten und der zweiten Absorptionsschicht ist eine Zwischenschicht eingefügt, die die erste und die zweite Absorptionsschicht voneinander trennt und eingerichtet ist, um

Flüssigkeit von der ersten zu der zweiten Absorptionsschicht zu leiten und auf die zweite Absorptionsschicht flächig zu verteilen. Die Zwischenschicht ist aus einem Vliesmaterial aus Baumwolle oder einem synthetischen baumwollartigen Material ausgebildet. Die Faserdicke, Faserlänge und die Vorzugsorientierung der Fasern des Vliesmaterials sind derart gewählt, dass eine Flüssigkeitsverteilung der aufgenommenen Flüssigkeit in einer Ebenenrichtung, in der Fläche gefördert wird und der Flüssigkeitstransport in der Ebenenrichtung höher oder stärker als derjenige in der Dickenrichtung ist.

Ohne an irgendeine Theorie gebunden sein zu wollen, ist festgestellt worden, dass kleinere SAP-Teilchen generell im Verhältnis zum Gewicht aufgrund der größeren Oberfläche in der Summe mehr Flüssigkeit aufnehmen können, aber schneller an ihre Kapazitätsgrenzen gelangen, was die Gefahr eines Reflux von Restflüssigkeit birgt. Deshalb werden hier größere SAP-Teilchen des Absorptionskerns in der ersten Absorptionsschicht verwendet, um eine Pufferfunktion zu erfüllen. Sie nehmen den ersten Flüssigkeitsschub sehr schnell und in großer Menge auf, ohne an ihre Kapazitätsgrenzen zu kommen. Verbleibende Flüssigkeitsmengen werden über die Zwischenschicht schnell abgeführt und breitflächig verteilt und an die zweite SAP-Schicht weitergeleitet. Die kleinen SAP-Teilchen in der zweiten Absorptionsschicht schließen die Flüssigkeit dauerhaft ein und geben sie auch unter Druck nicht ab, was den Reflux minimiert. Durch die Zusammenwirkung der Absorptionsschichten miteinander und mit der Zwischenschicht sowie mit weiteren Schichten, die die Flüssigkeit dem Absorptionskern zuführen, kann ein Absorptionsartikel mit sehr gutem Absorptionsvermögen bei relativ einfachem Aufbau und reduziertem Gewicht und Volumen des Absorptionskerns und des gesamten Absorptionsartikels erhalten werden. Der Absorptionskern bildet die Basis für sehr leistungsfähige, saug- und absorptionsfähige Einweg-Absorptionsartikel, die sich durch hohen Absorptionsgeschwindigkeiten und Absorptionskapazitäten, eine schnelle Trocknungszeit der Oberflächen und geringen Reflux auszeichnen. Das superabsorbierende Polymermaterial in den beiden Absorptionsschichten kann in Kombination das Zigfache, vorzugsweise wenigstens 50- oder 60-fache, seines eigenen Gewichts aufsaugen und unter Druck halten.

Der Absorptionsartikel ist bspw. für Windeln, insbesondere Babywindeln, Babywindelhöschen, Übungshöschen für Kleinkinder und Inkontinenzunterwünsche für Erwachsene zur Aufnahme und Absorption von Körperausscheidungen, wie Kot, Urin und anderer Körperflüssigkeiten, wie Blut, Vaginalausflüssen, Schweiß und dgl., gut geeignet. Der Absorptionskern ist auch für andere absorbierende Hygieneartikel, wie Binden, Stilleinlagen, Betteinlagen, Wundverbandprodukte oder dgl., vorteilhaft verwendbar.

Der Ausdruck "Einweg" wird hier in seinem üblichen Sinne verwendet und bedeutet einen Artikel, der nach einer geringen Anzahl von Verwendungen, bspw. weniger als 5 Verwendungen, vorzugsweise nach nur einer einzigen Verwendung, weggeworfen und entsorgt wird. Eine "Windel" bezeichnet einen Absorptionsartikel insbesondere für Säuglinge, Kleinkinder und Erwachsene, der um den unteren Torso einer Person herum gelegt und mittels eines Verschlusses fixiert wird, so dass er die Taille und die Beine des Trägers umschließt und im Einsatz Urin, Kot und andere Flüssigkeiten des Trägers aufnehmen und halten kann. Ein "Windelhöschen" hat die gleiche Funktion wie die Windel, ist jedoch mit einem durchgehenden Taillenband ausgestattet, so dass eine Taillenöffnung und zwei Beinöffnungen ausgebildet sind, durch die beim Anziehen des Windelhöschens die Beine des Trägers hindurchgesteckt werden, um das Windelhöschen an der Stelle des unteren Torso des Trägers zu platzieren. Ein Übungshöschen und sonstige Inkontinenzunterwäschen sollen von dem Begriff "Windelhöschen mit umfasst sein. Sofern in der nachstehenden Beschreibung auf eine Windel Bezug genommen wird, soll damit auch ein Windelhöschen oder ein beliebiger sonstiger vorstehend erwähnter vergleichbarer Absorptions- bzw. Hygieneartikel mit gemeint sein. "Teilchenförmig" in Verbindung mit dem SAP-Material soll hierin ein SAP-Material in Form von Granulat, kurzen Fasern, Plättchen, Flocken, Kugeln, Pulvern und anderen Formen bedeuten, die als Partikel, Teilchen oder Kerne bezeichnet werden können. Insbesondere können die SAP-Teilchen auch faserförmig, also etwas länglich ausgebildet sein. Das Verhältnis ihres Durchmessers zu ihrer Länge sollte jedoch nicht mehr als 1:10 und vorzugsweise weniger als 1:5 betragen. Als Teilchendurchmesser oder Kerndurchmesser eines Teilchens kann seine mittlere Dimension, also bspw. der Mittelwert aus Durchmesser und Länge eines kurzfaserigen SAP-Teilchens, bezeichnet werden. Der Teilchendurchmesser bzw. Kerndurchmesser des superabsorbierenden Polymermaterials in jeder der Absorptionsschichten ist als der der mittlere Teilchendurchmesser aller SAP-Teilchen in der jeweiligen Schicht zu verstehen.

In der Struktur des Absorptionskerns des Einweg-Absorptionsartikels gemäß der Erfindung ist die erste Absorptionsschicht an der körpernahen, inneren Seite, näher an der oberen Lage des Grundkörpers angeordnet, während die zweite Absorptionsschicht an der körperfernen, mehr außen befindlichen Seite, näher an der unteren Lage des Grundkörpers angeordnet ist.

Der Teilchendurchmesser des superabsorbierenden Polymermaterials in der zweiten Absorptionsschicht kann deutlich kleiner als derjenige in der ersten Absorptionsschicht sein. In bevorzugten Ausführungsformen beträgt der Teilchendurchmesser des SAP-Materials in der zweiten Absorptionsschicht weniger als 70%, vorzugsweise weniger als 60%, am meisten bevorzugt weniger als 50%, des Teilchendurchmessers des SAP-Materials in der ersten Absorptionsschicht. Ein kleinerer Teilchendurchmesser hilft, Wasser sicher zurückzuhalten und langfristig zu speichern. Die kleinen SAP-Polymerteilchen schließen jeweils die Flüssigkeit dauerhaft ein. Sie sind auch gegen Druck besonders unempfindlich, so dass sie den Reflux effektiv verhindern.

In besonders bevorzugten Ausführungsformen weist das superabsorbierende Polymermaterial der ersten Absorptionsschicht einen Teilchendurchmesser von etwa 0,595-0,400 mm auf, was einer Meshzahl von 30-40 entspricht, während das SAP-Material der zweiten Absorptionsschicht einen Teilchendurchmesser in einem Bereich von etwa 0,250-0,177 mm aufweist, was 60-80 Mesh entspricht.

Vorteilhafterweise kann in einem beliebigen vorstehend erwähnten Einweg-Absorptionsartikel das SAP-Material der ersten Absorptionsschicht eine 0,9% NaCl-Absorption von wenigstens 40 ml, vorzugsweise wenigstens 50 ml pro Gramm SAP und eine Frischwasserabsorption von wenigstens 80 ml, vorzugsweise wenigstens 100 ml pro Gramm SAP aufweisen. Das SAP-Material der zweiten Absorptionsschicht kann dann eine 0,9% NaCl-Absorption von wenigstens 60 ml, vorzugsweise wenigstens 65 ml pro Gramm SAP und eine Frischwasserabsorption von wenigstens 120 ml, vorzugsweise wenigstens 130 ml pro Gramm SAP aufweisen. In besonders bevorzugten Ausführungsformen beträgt die Frischwasserabsorption des SAP-Materials der zweiten Absorptionsschicht etwa 130-150 ml pro Gramm SAP. Es ist festgestellt worden, dass mit den vorstehend erwähnten Werten für Teilchendurchmesser und Wasserabsorptionsvermögen das beste Verhältnis von Leistung, betrachtet in Bezug auf Absorptionsgeschwindigkeit und -kapazität, zu Größe und Kosten des Absorptionskerns erreicht werden kann.

In vorteilhaften Ausführungsformen können die erste und die zweite Absorptionsschicht frei von Zellulose sein. Vorzugsweise kann der gesamte Absorptionskern zellulosefrei sein. Es hat sich gezeigt, dass durch Vermeidung des Einsatzes von Zellulose (auch als Zellstoff oder Pulp genannt), die als Absorptions- und Füllmaterial in Absorptionskernen herkömmlicher Absorptionsartikel weit verbreitet ist, das Absorptionsvermögen weiter verbessert werden kann, während gleichzeitig das Gewicht und die Größe des Absorptionskerns und des Absorptionsartikels insgesamt reduziert werden können.

In besonders bevorzugten Ausführungsformen kann das SAP-Material das einzige Absorptionsmaterial in der ersten und der zweiten Absorptionsschicht sein. Der Anteil von SAP-Material am gesamten Absorptionsmaterial in jeder der Absorptionsschichten beträgt somit ca. 100%; es ist kein anderes Absorptionsmaterial enthalten. Durch den Einsatz eines reinen SAP-Materials als Absorptionsmaterial in den Absorptionsschichten können das Absorptionsvermögen maximiert und dabei das Gewicht und die Größe des Absorptionskerns und des Absorptionsartikels minimiert werden. Es wird auch kein Klebstoff benötigt, der ansonsten erforderlich ist, um eine Trennung von SAP-Material vom Zellstoff oder von einem anderen Absorptions- oder Füllmaterial zu verhindern. Ein fehlender Klebstoff fördert wiederum das Absorptions- und Aufschwellvermögen des SAP-Materials.

Außerdem kann die Dicke des Absorptionskerns reduziert werden, was die Größe des Absorptionsartikels insgesamt verringert und den Tragekomfort verbessert. Die reduzierte Größe macht kleinere Verpackungen, insbesondere Großpackungen, weniger Stauraum für den Transport, die Lagerhaltung und im Verkaufsraum erforderlich und kann folglich Lager-, Transport- und Handhabungskosten einsparen. Es kann eine Volumeneinsparung von bis zu 20% gegenüber vergleichbaren herkömmlichen Absorptionsartikeln mit einem Pulp/SAP-Absorptionskern erzielt werden. Auch die anfallende Hausmüllmenge kann so deutlich verringert werden.

Das Gewicht des vorzugsweise auf reinem SAP-Material basierenden Absorptionskerns kann gegenüber vergleichbaren Pulp/SAP-Absorptionskernen gleicher Leistung wesentlich reduziert werden. In Realisierungen kann der Absorptionskern für eine Windel der Größe M/3 für ein Trägergewicht von 6-11 kg ein Kerngewicht von weniger als 20g, vorzugsweise weniger als 17g, am meisten bevorzugt etwa 15g, aufweisen. Bei einer Windel der Größe XXL/6 für ein Trägergewicht von 16-28 kg kann das Kerngewicht weniger als 25g, vorzugsweise weniger als 21g, betragen. Dies ergibt eine Gewichtsreduktion von bis 30-40% gegenüber vergleichbaren herkömmlichen Pulp/SAP-Absorptionskernen.

In bevorzugten Ausführungsformen eines beliebigen vorstehend erwähnten Einweg-Absorptionsartikels kann das superabsorbierende Polymermaterial in der ersten und zweiten Absorptionsschicht jeweils in Längsrichtung zwischen gegenüberliegenden Endrändern und in Seitenrichtung zwischen gegenüberliegenden Seitenrändern des Absorptionskerns kontinuierlich verteilt sein. Die Absorptionsschichten bilden folglich kontinuierliche, übereinanderliegende Schichten in Längs- und Seitenrichtung. Diskrete, voneinander getrennte Inselbereiche aus SAP-Material in den einzelnen Absorptionsschichten werden vermieden. Dies kommt auch einer verbesserten Diffusion der Flüssigkeit in den SAP-Absorptionsschichten zugute.

Die Zwischenschicht besteht aus einem Vlies aus einem Baumwollmaterial oder einem synthetischen baumwollartigen Material und weist eine Faserdicke, Faserlänge und Faserorientierung auf, die eine Flüssigkeitsverteilung der aufgenommenen Flüssigkeit in der Ebenenrichtung, also in Längs- und Seitenrichtung in der Ebene oder Fläche der Schicht, fördert. Ein "Vlies", hier auch als "Non-Woven-Material" bezeichnet, soll in der gesamten Beschreibung ein folien-, bahnen- oder mattenförmiges Produkt aus richtungsmäßig oder zufällig ausgerichteten Fasern bezeichnen, die durch Reibung, Kohäsion und/oder Adhäsion aneinander gebunden sind. Produkte, die gewebt, gestrickt, getuftet, durch Stiche unter Verwendung von Bindegarnen oder -fäden verbunden, oder durch Nassmahlen gefilzt sind, sollen nicht von dem Begriff "Vlies" oder "Non-Woven-Material" umfasst sein. Ein "baumwollartiges Material" soll hier ein Material bezeichnen, das im Wesentlichen die gleiche Faserlänge wie eine Baumwollfaser aufweist, und kann z.B. ein cotonisiertes Material oder ein Bikomponentenfasermaterial, bspw. auf Basis von Polyethylen (PE) und Polypropylen (PP), sein.

Die relativ dicke und dennoch leichte und elastische Vlieszwischenschicht sorgt für eine effektive Trennung der Absorptionsschichten voneinander, eine Stabilisierung derselben, eine breitflächige Flüssigkeitsverteilung und einen geringen Reflux. Ein Material hoher Faserlänge mit hoher Sperrigkeit und einer Vorzugsrichtung des Faserverlaufs in der Ebenenrichtung gegenüber der Dickenrichtung ist hierzu von Vorteil. Das Vliesmaterial der Zwischenschicht kann eine ums Mehrfache bessere Flüssigkeitsverteilung in der Fläche als vergleichbare Zellulosefasern haben. Dadurch wird die Benetzung des darunter liegenden SAP-Materials der zweiten Absorptionsschicht in der Fläche vergrößert, so dass dieses effektiver genutzt werden kann. Das SAP-Material kann über die Absorptionsschicht hinweg gleichmäßiger aufquellen.

In jedem beliebigen vorstehend erwähnten Einweg-Absorptionsartikel kann der Absorptionskern ferner eine Oberschicht, die eine erste, im Einsatz innerste, körpernahe, näher an der oberen Lage des Grundkörpers befindliche Schicht des Absorptionskerns bildet, und eine Unterschicht aufweisen, die eine letzte, im Einsatz äußerste, körperferne, näher an der unteren Lage des Grundkörpers befindliche Schicht des Absorptionskerns bildet. Die Oberschicht und die Unterschicht festigen, stabilisieren die gesamte Kernstruktur und vermeiden ein Verrutschen des Kerns in dem Grundkörper des Absorptionsartikels.

Die Oberschicht des Absorptionskerns kann insbesondere stark hydrophil, also wasseranziehend sein, um für einen schnellen Transport der Flüssigkeit in den Absorptionsteil des Kerns zu sorgen, so dass dieser Effekt auch bei hohen Flüssigkeitsmengen und längerer Beaufschlagung nicht unterbrochen wird. Die Oberschicht ist gemeinsam mit der Zwischenschicht so ausgelegt, dass sie das Wasser schnell transportieren und breitflächig verteilen können, um in Verbindung mit den beiden SAP-Schichten auf engstem Raum die Leistung zu verbessern. Die Oberschicht kann eine höhere Durchlässigkeit als die Unterschicht aufweisen. Die Unterschicht kann vorzugsweise hydrophob, also wasserabweisend sein.

Die Oberschicht und die Unterschicht bestehen vorzugsweise aus einem Vlies (Non-Woven-Material). Synthetische Non-Woven-Materialien, z.B. aus Polypropylenfaser oder Bikomponentenfaser, sind besonders geeignet. Alternativ können auch Vliesstoffe aus natürlichen Materialien, wie Seide, Woll- oder Baumwollfaser oder besonders bevorzugt Bambusfaser, zum Einsatz kommen. Damit kann auch eine biologisch abbaubare Windel geschaffen werden. Aufgrund der höheren Kosten und der begrenzten Steuerbarkeit der hydrophilen Eigenschaften mancher derzeit verfügbarer natürlicher Vliesstoffe werden synthetische Non-Woven-Materialien für die Ober- und Unterschicht derzeit bevorzugt. Die Ober- und Unterschicht sollten zudem eine hohe Reißfestigkeit haben, um den Kern auch im gefüllten Zustand stabil zu halten.

Bevorzugterweise kann die Unterschicht mit der Oberschicht nur an den Seitenrändern des Absorptionskerns verklebt sein. Dies in Verbindung mit der genügenden Eigenstabilität des Absorptionskerns ermöglicht diesem, noch fester in dem Absorptionsartikel, z.B. der Windel, zu liegen, so dass der Absorptionskern auch im nassen Zustand darin nicht verrutscht. Eine Fixierung der Ober- und Unterschicht oben und unten an den SAP-Schichten ist nicht erforderlich. Der Absorptionskern kann somit bis auf die Verklebungen an den Seitenrändern ansonsten frei von Haftmitteln zur Fixierung des SAP-Materials sein. Der Einsatz bedenklicher thermoplastischer Haftmittel kann vermieden werden. Dies ist für die Absorption von Vorteil. Außerdem zieht die Klebstoffeinsparung auch Kosteneinsparungen mit sich, weil haut- und umweltverträgliche Klebstoffe einen wesentlichen Kostenfaktor bei Windeln bilden. Es ist aber prinzipiell möglich, die Ober- und Unterschicht alternativ mit speziellen toxisch und ökologisch unbedenklichen, umwelt- und hautverträglichen Klebstoffen mit der jeweiligen SAP-Schicht teilflächig zu verkleben.

In vorteilhaften Ausführungsformen kann jeder beliebige vorstehend erwähnte Einweg-Absorptionsartikel ferner eine Aufnahme- und Verteilungsschicht (sog. ADL-Schicht, Acquisition Distribution Layer) aufweisen, die zwischen der oberen Lage des Grundkörpers und dem Absorptionskern eingefügt und eingerichtet ist, um aufgenommene Flüssigkeit schnell breitflächig an den Absorptionskern weiterzuleiten. Derartige ADL-Schichten sind aus dem Stand der Technik bekannt. Die ADL-Schicht in dem erfindungsgemäßen Einweg-Absorptionsartikel weist jedoch vorzugsweise eine grobfaserige Struktur aus einem Vlies (Non-Woven-Material), bspw. ähnlich der Zwischenschicht in dem Kern, auf, um die Flüssigkeit breit in der Fläche, möglichst auf die gesamte verfügbare Fläche des Absorptionskerns zu verteilen. Es sind auch andere Materialien als Vliesstoffe für die ADL-Schicht verwendbar.

Vorzugsweise sind die obere Lage und die ADL-Schicht mit dem Absorptionskern nicht verklebt. Damit wird eine Ausdehnung des Absorptionskerns bei Flüssigkeitsaufnahme nicht behindert. Eine Stabilität der Gesamtwindel kann durch geeignete Dimensionen der Komponenten und Fertigungstechniken sichergestellt werden. Wiederum können ökologisch bedenkliche Kleber vermieden und Kosten eingespart werden.

In einer vorteilhaften Weiterbildung eines beliebigen vorstehend erwähnten Einweg-Absorptionsartikels kann die untere Lage des Grundkörpers durch eine Vliesaußenschicht gebildet sein. Zwischen der Vliesaußenschicht und dem Absorptionskern kann eine Auslaufschutzschicht in Form einer flüssigkeitsundurchlässigen Folie angeordnet sein. Die flüssigkeitsundurchlässige Folie kann vorzugsweise aus Polyethylen (PE) oder Polypropylen (PP) oder ähnlichen Materialien bestehen. Zur Verringerung der Pilzbildung und zur Verhinderung eines Wärmestaus in dem Absorptionsartikel wird eine atmungsaktive Folie bevorzugt. Eine weiche Vliesaußenschicht kann aus demselben Material wie die Ober-, Unter- und/oder Zwischenschicht des Absorptionskerns bestehen.

In einer Ausführungsform kann die obere Lage des Grundkörpers bis zu der Auslaufschutzschicht des Grundkörpers herangeführt und dort schmalflächig mit dieser verklebt sein. Damit kann eine Umhüllung für den Absorptionskern geschaffen werden, die diesem guten strukturellen Halt bietet.

In einer modifizierten Ausführungsform kann die obere Lage noch weiter verlängert und umgeschlagen sein, so dass sie mit ihren Randabschnitten den Absorptionskern an den beiden lateralen Seiten bedeckt und von unten, auf der Seite der Unterschicht des Kerns geringfügig umgreift. Vorzugsweise können diese Randabschnitte zusätzlich mit der Auslaufschutzschicht des Grundkörpers schmalflächig verklebt sein. Dies ermöglicht es, Ausscheidungsflüssigkeit, die von dem Absorptionskern nicht aufgenommen oder wieder abgegeben wird, auch im Bereich der Auslaufschutzschicht bzw. der unteren Lage zu absorbieren. Außerdem wird durch diese Ausgestaltung der strukturelle Halt des Absorptionskerns in dem Grundkörper weiter verbessert.

In jeder der vorstehend erwähnten Ausführungsform des Einweg-Absorptionsartikels kann der Absorptionskern sehr gutes Absorptionsverhalten, sowohl hinsichtlich der Absorptionsgeschwindigkeit als auch hinsichtlich der Absorptionskapazität, ein reduziertes Gewicht, eine reduzierte Größe, eine schnelle Trocknungszeit der Oberfläche und einen minimierten Reflux ermöglichen. Dies wird insbesondere durch die abgestimmte Auswahl der Materialien des Kerns, der SAP-Materialien mit unterschiedlichen Teilchendurchmessern in den Absorptionsschichten und der Zwischenschicht zwischen diesen gefördert. Die vorteilhaften Eigenschaften kommen auch dem Absorptionsartikel, insbesondere einer Windel oder einem Windelhöschen, insgesamt zugute.

Es kommen auch alternative Materialien für den Absorptionskern und den Absorptionsartikel in Frage. Insbesondere können nachwachsende Rohstoffe verwendet werden, um eine vollständig kompostierbare Windel zu schaffen. Bspw. können die Superabsorber aus Stärke gewonnen werden. Als Auslaufschutzschicht können biologisch abbaubare Materialien verwendet werden. Die Non-Woven-Schichten können auf Bambusfaserstoffen oder speziell behandelter Zellulose basieren. Für die Zwischenschicht des Absorptionskerns kann Naturbaumwolle verwendet werden.

Weitere vorteilhafte Einzelheiten und besondere Merkmale und Eigenschaften der Erfindung erschließen sich aus der folgenden Beschreibung bevorzugter Ausführungsformen der Erfindung, den beigefügten Zeichnungen und den Patentansprüchen. In den Zeichnungen sind Ausführungsformen der Erfindung lediglich zu Beispiels- und Veranschaulichungszwecken und nicht zur Beschränkung der Erfindung angegeben, wobei gleiche Bezugszeichen gleiche Elemente überall in den Zeichnungen bezeichnen. Es zeigen:
Fig. 1 eine Draufsicht auf eine Windel gemäß einer Ausführungsform der Erfindung, in vereinfachter Darstellung, teilweise in mehreren Ebenen aufgeschnitten;
Fig. 2 einen Querschnitt durch die Windel nach Fig. 1, aufgenommen entlang der Linie 2-2 in Fig. 1;
Fig. 3 eine Querschnittsansicht eines Absorptionskerns der Windel nach Fig. 1 in einer isolierten, vereinfachten, vergrößerten Darstellung;
Fig. 4 einen Querschnitt durch die Struktur der Windel nach Fig. 1 unter Darstellung der Flüssigkeitsabsorption zur Veranschaulichung der Funktionsweise, in einer stark vereinfachten, gegenüber Fig. 1 vergrößerten Darstellung; und
Fig. 5 eine Windel gemäß einer weiteren Ausführungsform der Erfindung, in vereinfachter Querschnittsdarstellung, ähnlich der Darstellung nach Fig. 2.

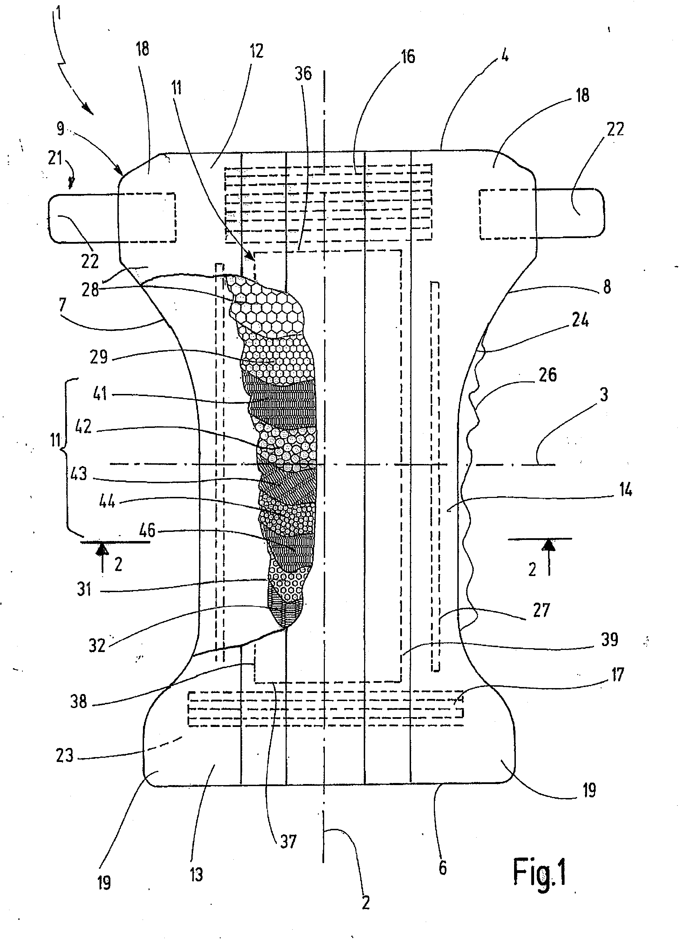

In Fig. 1 ist eine Windel 1 gemäß einer Ausführungsform der Erfindung in Draufsicht, in vereinfachter Darstellung veranschaulicht. Die Windel 1 stellt ein Beispiel für einen Einweg-Absorptionsartikel dar, der die Erfindung verkörpert. Wenngleich im Folgenden insbesondere auf die Windel 1 nach Fig. 1 Bezug genommen wird, versteht es sich, dass die Erfindung auch auf andere Einweg-Absorptionsartikel, insbesondere Windelhöschen für Säuglinge, Übungshöschen für Kleinkinder und Inkontinenzunterwäsche für Erwachsene, angewandt werden kann und die nachstehenden Ausführungen für diese Absorptionsartikel analog gelten. Bei der Windel 1 oder einem sonstigen Absorptionsartikel handelt es sich hier um einen Einweg- oder Wegwerfartikel, der nach wenigen Verwendungen, vorzugsweise nach nur einer einzigen Verwendung weggeworfen oder entsorgt wird. Die Windel 1 ist nicht zur mehrmaligen Wiederverwendung bestimmt.

Die Windel 1 ist in Fig. 1 in einem ausgebreiteten, nicht zusammengezogenen Zustand dargestellt, wobei Bereiche des Aufbaus teilweise weggeschnitten sind, um den Innenaufbau der Windel 1 klarer zu zeigen. Die im Einsatz körperseitige Oberfläche der Windel, welche mit der Haut des Trägers in Kontakt gelangt, ist in Fig. 1 dem Betrachter zugewandt. Dargestellt sind auch eine Längsachse 2 der Windel 1 und eine hierzu senkrechte, in Seitenrichtung verlaufende Querachse 3. Die äußeren Grenzen der Windel definieren einen Außenrand, zu dem ein in Fig. 1 oberer Rand 4 und ein gegenüberliegender unterer Rand 6, die die Windel in Längsrichtung entlang der Längsachse 2 begrenzen, sowie ein in Figur 1 linker und rechter Rand 7, 8 gehören, die in Längsrichtung im Wesentlichen parallel zu der Längsachse 2 verlaufen und die äußeren Begrenzungsränder in Seitenrichtung längs der Querachse 3 definieren. Es versteht sich, dass die Ausdrücke "oberer", "unterer", "linker" und "rechter", wie sie hier in Verbindung mit den Rändern 4-8 oder auch mit anderen Komponenten der Windel verwendet werden, lediglich der Vereinfachung der Bezugnahme auf die Windel 1 und deren Komponenten dienen und von der Orientierung der Windel abhängig sind. Diese Ausdrücke beziehen sich hier auf die in Fig. 1 dargestellte Orientierung der Windel 1.

Die Windel 1 weist einen Grundkörper 9 und einen Absorptionskern 11 auf. Der Grundkörper 9 bietet der Windel 1 strukturellen Halt und nimmt den Absorptionskern 11 in seinem Inneren stabil auf. Der Grundkörper 9 bzw. die Windel 1 weist einen ersten oder hinteren Bundabschnitt 12 auf, der im Einsatz die hintere Taillenregion des Trägers umgibt, einen zweiten oder vorderen Bundabschnitt 13, der im Einsatz die vordere Taillenregion des Trägers umgibt, und einen Zwischenabschnitt 14 auf, der den hinteren und vorderen Bundabschnitt 12, 13 verbindet. Der Zwischenabschnitt 14 ist im Einsatz im Schrittbereich eines Trägers, zwischen dessen Beinen angeordnet. Der hintere Bundabschnitt 12 kann ein eingebettetes elastisches Bündchenelement 16 aufweisen, das es ermöglicht, den Bundabschnitt 12 in Seitenrichtung elastisch zu strecken, so dass es ein Anbringen der Windel 1 um die Taille des Trägers erleichtert und es sich zusammenziehen kann, um eine genaue Passform und gute Dichtigkeit im Einsatz zu fördern.

Der Zwischenabschnitt 14 weist die geringste seitliche Ausdehnung auf. Der hintere und vordere Bundabschnitt 12, 13 sind breiter als der Zwischenabschnitt 14 und definieren jeweils ein Paar flügelartiger hinterer Randabschnitte 18 bzw. flügelartiger vorderer Randabschnitte 19, die in Seitenrichtung über den Zwischenabschnitt 14 vorragen. An den hinteren Randabschnitten 18 sind Befestigungselemente 22 eines Befestigungssystems 21 zur lösbaren und wiederverschließbaren Befestigung der Windel 1 um die Taille des Trägers vorgesehen. Die Befestigungselemente 22 können in einfachster Form als Befestigungsbänder nach Art von Pflastern ausgebildet sein, die bei Befestigung der Windel 1 um die Taille des Trägers an einen Anlagebereich 23, der an der Außenfläche des vorderen Randabschnitts 19 vorgesehen ist, angelegt und dort durch Adhäsions- oder Kohäsionskraft gehalten werden. In der hier veranschaulichten bevorzugten Ausführungsform ist ein mechanisches Befestigungssystem 21 nach Art eines Klettverschlusses vorgesehen, bei dem die Befestigungselementen 22 bspw. als Hakenbänder ausgebildet sind, die mit einem Schlaufen- oder Flauschband 17 zusammenwirken, das in dem Anlagebereich 23 an der Au-ßenseite der Windel 1 angebracht, z.B. angeklebt ist. Damit kann die Windel 1 nahezu beliebig oft geöffnet und verschlossen werden. Es sind noch andere wiederholt lösbare Verschlussmittel, wie bspw. Haken- und Ösen-Mechanismen oder dgl., möglich.

Jedenfalls ermöglicht das Befestigungssystem 21 in Kombination mit dem elastischen Bündchenelement 16 in dem Bundabschnitt 12 eine einfache Anordnung und Sicherung der Windel 1 um die Taille des Trägers. Im befestigten Zustand nehmen die Befestigungselemente 22 eine Zuglast um die Taille des Trägers herum auf. An dem Anlagebereich 23, insbesondere dem Schlaufen- oder Flauschband 17, können zu beiden Seiten in Querrichtung geeignete (hier nicht veranschaulichte) Markierungen vorgesehen sein, die dem Benutzer helfen können, die Windel 1 gleichmäßig um die Taille zu schließen und die Zuglast gleichmäßig auf beide Befestigungselemente 22 zu verteilen.

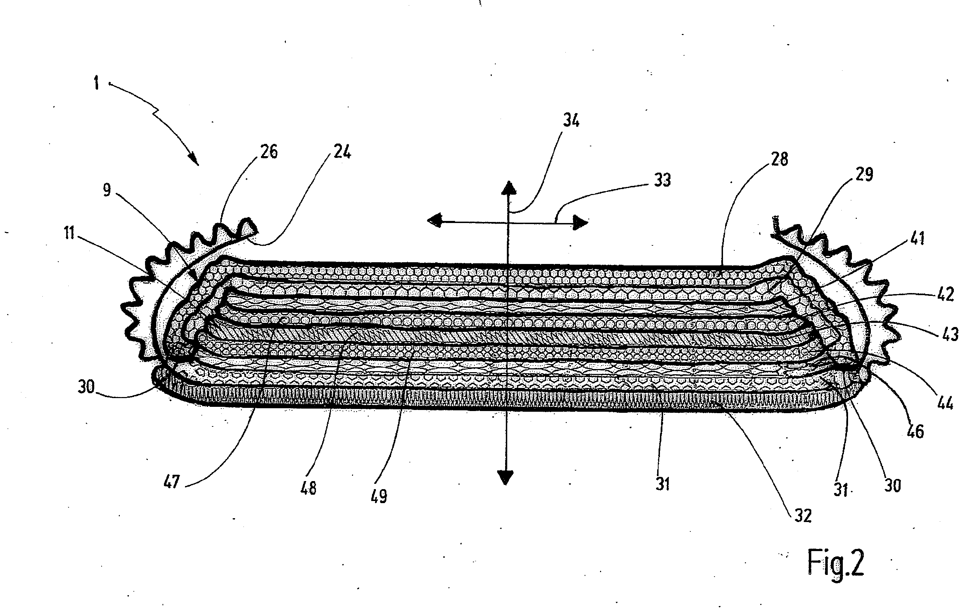

In dem Zwischenabschnitt 14 sind entlang des linken und des rechten Randes 7, 8 zwei elastische Reihen von Beinbündchen 24, 26 vorgesehen, die im Einsatz das Bein des Trägers dichtend umschließen. Die elastischen Bündchen 24, 26 gehen auch aus der vergrößerten Querschnittsansicht der Windel 1 in Fig. 2 deutlich hervor. Das innere Bündchen 24 liegt eng am Bein an und stellt eine erste Barriere gegen Austritt von Flüssigkeit dar. Das äußere Bündchen 26 bildet eine zweite Barriere und kann vorteilhafterweise in Rüschenform ausgebildet sein. Elastische Elemente 27, bspw. in den Grundkörper 9 eingenähte elastische Fäden 27, unterstützen die Elastizität, Passform und Dichtigkeit der Bündchen 24, 26.

Wie aus dem weggeschnittenen Abschnitt in Fig. 1 und der Querschnittsdarstellung nach Fig. 2 ersichtlich, weist der Grundkörper 9 eine Schichtstruktur auf, zu der eine obere Lage 28, eine Aufnahme- und Verteilungsschicht 29, eine Auslaufschutzschicht 31 und eine untere Lage 32 gehören. Die obere Lage 28, die in Figur 1 sowohl in Draufsicht als auch aufgeschnitten dargestellt ist, ist die Schicht, die im Einsatz mit der Haut des Trägers in direktem Kontakt steht. Sie ist aus einem äußerst weichen Material, vorzugsweise Vliesstoff ausgebildet, um einen hohen Tragekomfort zu gewährleisten. Unter "Vliesstoff", oftmals und hier auch als "Non-Woven-Material" bezeichnet, wird hier ein folien-, bahn- oder mattenförmiges Produkt aus richtungsmäßig oder zufällig ausgerichteten Fasern verstanden, die durch Reibung, Kohäsion und/oder Adhäsion aneinander gebunden und nicht gewebt, gestrickt, getuftet oder durch Stiche miteinander verbunden sind. Die in dem Schichtaufbau der Windel 1 verwendeten Non-Woven-Vliesstoffe können aus verschiedenen natürlichen oder synthetischen Fasern bestehen und können gestapelte oder kontinuierliche Filamente sein. Sie können durch verschiedene Verfahren, wie Schmelzblasen, Spunbonden, Spinnen oder Kardieren und andere, hergestellt werden. Synthetische Fasern werden derzeit bevorzugt. Es sind aber auch Vliesstoffe aus natürlichen Materialien, wie z.B. Seide und Woll- oder Baumwollfasern, oder bevorzugt aus Bambusfasern, die gute Saugfähigkeiten zeigen, verwendbar. Prinzipiell sind auch synthetische und natürliche Mischmaterialien verwendbar.

Obwohl dies in den Figuren nicht gesondert dargestellt ist, weist die obere Lage 28 vorzugsweise eine noppenförmige Oberflächenstruktur mit einer Vielzahl verteilter perlenförmiger Noppen oder Erhebungen, die von der Außenoberfläche der oberen Lage 28 nach außen vorstehen. Aufgrund ihrer Perlenform werden derartige punktartige Noppen auch als "Pearl Dot" bezeichnet. Diese Noppenstruktur ermöglicht einen schnelleren Abfluss der von einem Träger ausgeschiedenen und von der oberen Lage 28 absorbierten Flüssigkeit in die darunter liegende Aufnahme- und Verteilungsschicht 29. Außerdem verringern die Noppen auch den Hautkontakt, wodurch sie den Tragekomfort erhöhen, Hautreizungen reduzieren und eine Belüftung der Haut des Trägers fördern. Alternativ kann zu diesem Zweck auch eine sehr weiche, leicht perforierte obere Lage 28 verwendet werden, die bspw. über einpresste Kanäle verfügen kann. Die obere Lage 28 ist hydrophil ausgelegt, um eine schnelle Flüssigkeitsableitung sicherzustellen. Insbesondere für Babywindeln sollte ein besonders weiches Vlies mit einer vergleichbaren Haptik von Baumwolle verwendet werden.

Die Aufnahme- und Verteilungsschicht 29 ist zwischen der oberen Lage 28 und dem Absorptionskern 11 angeordnet. Sie wird häufig auch als ADL-Schicht (Acquisition Distribution Layer) bezeichnet und hat die Funktion, die von der oberen Lage 28 aufgenommene Ausscheidungsflüssigkeit schnell in den darunter liegenden Absorptionskern 11 zu transportieren und dabei die Flüssigkeit möglichst breit in der Fläche zu verteilen, um eine große zur Verfügung stehende Fläche des Absorptionskerns 11 zur Absorption zu nutzen. Die ADL-Schicht 29 ist hierzu aus einem geeigneten Material, wie bspw. einem Vlies, also einem Non-Woven-Material, wie oben erläutert, ausgebildet, das besonders grobe Fasern aufweist, die eine geeignete Vorzugsorientierung des Faserverlaufs aufweisen können, um die Flüssigkeitsverteilung in der Ebenenrichtung 33, also in der Fläche zu fördern, dabei aber einen schnellen Abtransport der Ausscheidungsflüssigkeit in der Tiefen- bzw. Dickenrichtung 34 aufrechtzuerhalten.

Die untere Lage 32 des Grundkörpers 9 bildet die Außenschicht des Grundkörpers 9 und der Windel 1 und hat die Funktion, die Gesamtstruktur zu halten. Sie kann aus ähnlichem oder gleichem Material, insbesondere synthetischem Non-Woven-Material (Vliesstoff), wie die obere Lage 28 (jedoch ohne eine Noppenstruktur oder dgl.) gebildet sein. Ein besonders angenehmes, weiches Material ist insbesondere, aber nicht nur für Babywindeln erwünscht.

Da die Außenschicht 32 unmittelbar mit der Kleidung des Trägers oder mit anderen Artikeln, wie bspw. Bettwäsche, in Kontakt gelangt, muss verhindert werden, dass Ausscheidungen, die in dem Absorptionskern absorbiert und gehalten werden, zur Außenfläche gelangen und die Kleidung, Bettwäsche oder dgl. verunreinigen. Hierzu ist die Auslaufschutzschicht 31 vorgesehen, die eine flüssigkeitsundurchlässige Folie ist, die vorzugsweise aus Polyethylen (PE), Polypropylen (PP) oder anderen geeigneten Materialien hergestellt ist, die die Fluiddichtigkeit sicherstellen können. Geeignete Auslaufschutzschichten oder -folien 31 sollten atmungsaktiv sein, um ein Eindringen von Luft ins Innere der Windel 1 zur Belüftung der Haut des Trägers zuzulassen und die Flüssigkeit diffundieren zu lassen. Eine Atmungsfähigkeit der Auslaufschutzschicht 31 kann so Wärmestau und Pilzbildung verhindern. Die Stärke der Auslaufschutzschicht 31 muss den Anforderungen entsprechend angemessen gewählt werden, wobei sich bspw. PE-Folien mit einer Dicke von etwa 15-16 g/qm als passend erwiesen haben. Di Folie kann auch bedruckt sein.

Wie insbesondere aus Fig. 2 ersichtlich, ist die obere Lage 28 entlang der Seitenränder des Grundkörpers 9, an der ADL-Schicht 29 und dem Absorptionskern 11 vorbei, bis zu der und der Auslaufschutzschicht 31 fortgeführt und dort mit der und der Auslaufschutzschicht 31 und gegebenenfalls auch mit dem inneren Bündchen 24 an einer Stelle 30 flächig verklebt. Die obere Lage 28 kann dann Ausscheidungsflüssigkeit, wie z.B. Urin, die aus der Schicht 29 oder dem Absorptionskern 11 zur Seite hin austritt auffangen und absorbieren und so in Kombination mit den Bündchen 24, 26 für eine erhöhte Dichtigkeit sorgen. Außerdem ist so ein geschlossener Grundkörper 9 geschaffen, der den Absorptionskern 11 umgibt und stabil aufnimmt und auch der gesamten Windel 1 guten strukturellen Halt bietet.

Der Grundkörper 9 der Windel 1 weist eine für eine jeweilige Trägeralters- und -gewichtsgruppe geeignete dreidimensionale Gestalt auf, die für eine gute Passform und einen guten Tragekomfort ausgelegt ist. Die weichen Materialien, die mit der Haut des Trägers in Kontakt gelangen oder die Außenseite der Windel 1 bilden, vermitteln ein äußerst angenehmes, weiches haptisches Gefühl. Der Grundkörper 9 ist zur Aufnahme und zum stabilen Halt des Absorptionskerns 11 in seinem Inneren gut geeignet.

Der Absorptionskern 11 bildet die Hauptkomponente der Windel 1, die für die Absorption und Zurückhaltung der Ausscheidungsflüssigkeit sorgt. Der Absorptionskern 11 ist im Inneren des Grundkörpers 9 zwischen der ADL-Schicht 29 und der Auslaufschutzschicht 31 eingefügt und weitgehend verrutchfest gehalten. Der Absorptionskern 11 weist hier in Draufsicht gemäß Fig. 1 eine im Wesentlichen rechteckige Kontur auf, die etwas kleiner als diejenige des Grundkörpers 9 ist. Die Kontur des Absorptionskerns 11 ist durch einen in Fig. 1 oberen Rand 36 und einen gegenüberliegenden unteren Rand 37, die den Absorptionskern 11 in Längsrichtung entlang der Längsachse 2 begrenzen, sowie einen in Figur 1 linken und rechten Seitenrand 38, 39 definiert, die in Längsrichtung im Wesentlichen parallel zu der Längsachse 2 verlaufen. Die Länge des Absorptionskerns 11 entlang der Längsachse 2 entspricht im Wesentlichen der Längsausdehnung des Zwischenabschnitts 14 des Grundkörpers 9, und die Breite des Absorptionskerns 11 ist etwas kleiner als die Breite des Zwischenabschnitts 14. Die Kontur des Absorptionskerns 11 könnte an die Kontur des Zwischenabschnitts 14 angepasst sein, und die Seitenränder 38, 39 könnten dem Verlauf der Ränder 7 bzw. 8 des Grundkörpers 9 entsprechend leicht gekrümmt sein und in geringem Abstand zu diesen verlaufen.

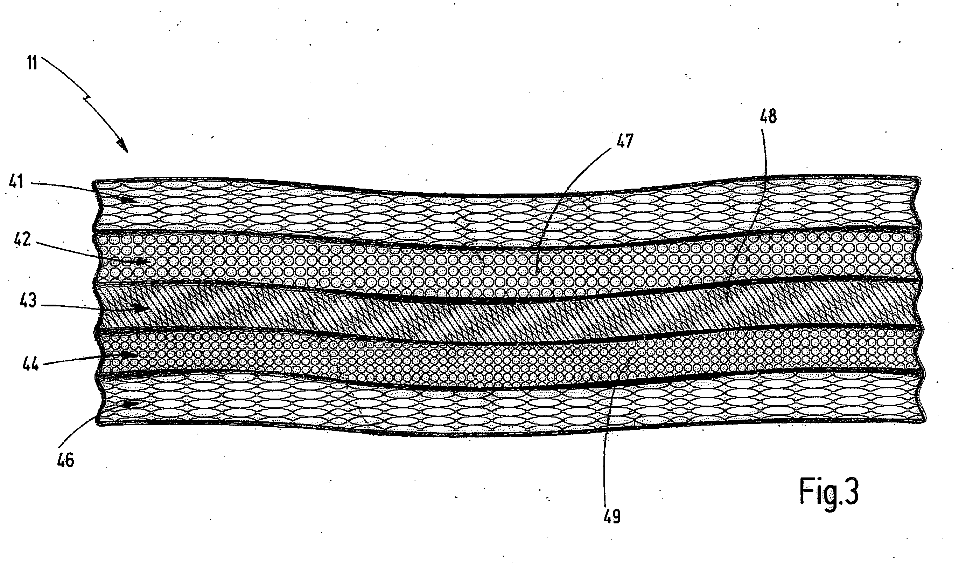

Der Absorptionskern 1 ist zusätzlich in Figur 3 in einer vergrößerten isolierten Darstellung veranschaulicht. Er weist eine Schichtstruktur und ein Absorptionsmaterial auf, die speziell gestaltet, ausgewählt und aufeinander abgestimmt sind, um ein möglichst gutes Absorptionsverhalten in Bezug auf sowohl die Absorptionsgeschwindigkeit als auch die Absorptionskapazität bei möglichst geringem Gewicht des Kerns 11 zu ermöglichen und einen Rückfluss (Reflux) von Flüssigkeit unter Druck zu minimieren. Der Absorptionskern 11 umfasst nicht die Schichten des Grundkörpers 9, insbesondere nicht die obere Lage 28 oder die ADL-Schicht 29 auf der körpernahen Seite und auch nicht die Auslaufschutzschicht 31 oder die äußere, untere Lage 32 auf der körperfernen Seite der Windel 1.

Vielmehr gehört zu der Schichtstruktur des Absorptionskerns 11 hier eine Oberschicht 41, eine erste Absorptionsschicht 42, eine Zwischenschicht 43, eine zweite Absorptionsschicht 44 und eine Unterschicht 46. Die angegebene Reihenfolge der Schichten 41-46 gilt in Dicken- bzw. Tiefenrichtung 34 des Absorptionskerns 11 bzw. der Windel 1.

Die Oberschicht 41 bildet die oberste Kernschicht, die in Dickenrichtung 34 unterhalb der bzw. angrenzend an die Aufnahme- und Verteilungs(ADL)-Schicht 29 des Grundkörpers 9 angeordnet ist. Die Oberschicht 41 ist hydrophil, also wasseranziehend, um für einen schnellen Abtransport der Ausscheidungsflüssigkeit von der ADL-Schicht 29 in den Absorptionsteil des Absorptionskerns 11 zu sorgen. Dieser Abtransport muss auch bei hohen Flüssigkeitsmengen gewährleistet sein. Deshalb weist die Oberschicht 41 vorzugsweise eine höhere Durchlässigkeit in Dickenrichtung als die darüber liegenden Schichten 28, 29 des Grundkörpers 9 auf. Es sei bemerkt, dass die ADL-Schicht 29 des Grundkörpers 9 auch weggelassen werden könnte, insbesondere wenn die Oberschicht 41 des Absorptionskerns 11 so ausgelegt ist, dass sie die Aufnahme- und Verteilungsfunktion der ADL-Schicht 29 mit erfüllt.

Außerdem dient die Oberschicht 41 dazu, die gesamte Struktur des Absorptionskerns 11 zu festigen und ein Verrutschen desselben in dem Grundkörper 9 der Windel 1 zu vermeiden. Ferner sorgt sie für eine Stabilität des Absorptionskerns 11 auch während des Fertigungsprozesses. Die Oberschicht 41 besteht vorzugsweise aus einem synthetischen Non-Woven-Material (Vlies), das dem Material der oberen bzw. unteren Lage 28, 32 des Grundkörpers 9 ähnlich oder mit diesem/diesen identisch sein kann. Als geeignet hat sich ein Vliesmaterial bspw. aus Polypropylen (PP) mit einem Gewicht von etwa 11-13 g/qm erwiesen. Das Vlies der Oberschicht 41 kann, muss aber nicht so weich wie das die Haut berührende Material der oberen Lage 28 des Grundkörpers 9 sein.

An die Oberschicht 41 schließt sich in Dickenrichtung 34 die erste Absorptionsschicht 42 an. Die Absorptionsschicht 42 weist in der bevorzugten Ausführungsform ein teilchenförmiges, superabsorbierendes Polymermaterial 47 auf, das einen ersten, großen Teilchendurchmesser aufweist. Unter "superabsorbierendem Polymermaterial" ("SAP-Material"), "Superabsorber" oder auch "gelierendem Absorptionsmaterial" wird auf dem Gebiet und hierin austauschbar ein vernetztes Polymermaterial bezeichnet, das mindestens das 10-fache seines Gewichtes an Frischwasser absorbieren kann, wobei es bei Aufnahme von Wasser aufquillt oder geliert. Im Allgemeinen wird für ein SAP-Material ein Copolymer aus Akrylsäure (Propensäure) oder Natriumacrylat (Natriumsalz der Akrylsäure) einerseits und Akrylamid andererseits verwendet, wobei das Verhältnis der beiden Monomere zueinander variieren kann. Zusätzlich wird ein sog. Kernvernetzer (Core Cross-Linker CXL) der Monomerlösung zugesetzt, der die gebildeten langkettigen Polymermoleküle durch chemische Brücken stellenweise untereinander verbindet bzw. vernetzt. Durch diese Brücken wird das Polymer wasserunlöslich. Dieses sog. Basispolymer wird gegebenenfalls einer Oberflächen-Nachvernetzung (Surface Cross-Linking, SXL) unterzogen. Dabei wird eine weitere Chemikalie auf die Oberfläche der Partikel aufgebracht, die durch Erwärmung ein zweites Netzwerk nur auf der äußeren Schicht des Korns bildet. Diese Hülle stützt das nach Aufnahme von Flüssigkeit aufgequollene Gel, um dieses auch bei äußerer Belastung durch Bewegung und Druck zusammenzuhalten. Dies ermöglicht es, die in dem SAP-Material absorbierte Flüssigkeit auch für längere Dauer, für 12 Stunden oder mehr, sicher zu halten, ohne dass es zu einem Rückfluss kommt.

"Teilchenförmiges" SAP-Material soll hierin ein SAP-Material in Form von Granulat, kurzen Fasern, Plättchen, Flocken, Kugeln, Pulvern und anderen Formen bedeuten, die als Partikel, Teilchen oder Kerne bezeichnet werden können und deren Verhältnis ihrer Breite oder ihres Durchmesser zu ihrer Länge weniger als 1:10, vorzugsweise weniger als 1:5 beträgt. Als Teilchen- oder Kerndurchmesser eines SAP-Partikels wird seine mittlere Dimension, also bspw. der Mittelwert aus Breite, Dicke (oder Durchmesser) und Länge, bezeichnet. Der Teilchendurchmesser bzw. Kerndurchmesser des SAP-Materials in jeder der Absorptionsschichten 42, 44 ist als der Mittelwert der Teilchendurchmesser aller SAP-Teilchen in der jeweiligen Schicht zu verstehen.

Die erste Absorptionsschicht 42 ist ausgewählt, um eine Art Pufferfunktion zu erfüllen. Sie soll den ersten Flüssigkeitsschub relativ schnell von der Oberschicht 41 übernehmen und in großer Menge aufnehmen und nicht an ihre Absorptionskapazitätsgrenze gelangen. Hierzu ist der Teilchendurchmesser bzw. Kerndurchmesser des teilchenförmigen SAP-Materials 47 der ersten Absorptionsschicht 42 geeignet groß (insbesondere größer als derjenige eines SAP-Materials der zweiten Absorptionsschicht 44, wie nachstehend näher beschrieben) gewählt, um diese Funktion zu erfüllen. Als geeigneter erster Teilchendurchmesser für das teilchenförmige SAP-Material 47 der ersten Absorptionsschicht 42 hat sich ein Durchmesser im Bereich von 0,595-0,400 mm erwiesen, der 30-40 Mesh entspricht. Der Teilchendurchmesser könnte aber je nach SAP-Material 47 geringfügig nach oben oder unten variieren. Das SAP-Material 47 der ersten Absorptionsschicht 42 sollte aber eine 0,9% NaCl-Absorption von wenigstens 40 ml, vorzugsweise wenigstens 50 ml pro Gramm SAP aufweisen. Die Frischwasserabsorption sollte nicht unter 80 ml pro Gramm SAP liegen und vorzugsweise wenigstens 100 ml pro Gramm SAP betragen. Derartige Leistungswerte können von hochqualitativen Superabsorbern erreicht werden, die bspw. von den Firmen BASF SE, Deutschland, Sanyo Chemical Industries Ltd., Japan, unter dem Handelsnamen Sandia oder von Evonic Industries AG, Deutschland, unter dem Handelsnamen Favor kommerziell erhältlich sind.

Das SAP-Material 47 in der ersten Absorptionsschicht 42 ist vorzugsweise das einzige Absorptionsmaterial in dieser Schicht. In anderen Worten beträgt der Anteil des SAP-Materials 47 an dem gesamten Absorptionsmaterial in der ersten Absorptionsschicht 42 also 100% oder wenigstens nahezu 100%. Jedenfalls ist keine Zellulose (Pulp) in dem Absorptionsmaterial der ersten Absorptionsschicht 42 enthalten. Es ist festgestellt worden, dass mit einem Absorptionsmaterial, das auf reinem SAP-Material basiert, die beste Leistung in Bezug auf eine schnelle Aufnahme der Flüssigkeit und eine große Absorptionsmenge und auch hinsichtlich einer schnellen Trocknungszeit einer Oberfläche bei geringem Gewicht und Volumen des Absorptionskerns 11 erreicht werden kann. Letzteres ist insbesondere darauf zurückzuführen, dass das Absorptionsmaterial zellulosefrei ist. Außerdem können Probleme, die bei herkömmlichen SAP/Pulp-Absorptionskernen auftreten können, wie bspw. eine ungleichmäßige Verteilung des SAP-Materials in dem Pulp mit stärkeren und schwächeren Absorptionsbereichen, ein Verrutschen des Kerns in dem Grundkörper, usw., vermieden werden. Es kann ein gutes und gleichmäßiges Absorptionsvermögen und Aufschwellvermögen des SAP-Materials über den gesamten Absorptionskern 11 hinweg bei reduziertem Gewicht und Volumen des Kerns 11 erzielt werden. Im Allgemeinen genügt für eine Windel der Größe M/3, die für einen Träger mit einem Körpergewicht von etwa 6-11 kg bestimmt ist, etwa 6-7 g des SAP-Materials in der ersten Absorptionsschicht 42.

Der SAP-Teilchendurchmesser für die erste Absorptionsschicht 42 wird gezielt so groß gewählt, weil festgestellt wurde, dass kleinere SAP-Partikel zwar wegen der größeren Oberfläche generell im Verhältnis zum Gewicht in der Summe mehr Flüssigkeit aufnehmen können, jedoch schneller an ihre Kapazitätsgrenzen kommen. Ein zu geringe Teilchendurchmesser in der ersten Absorptionsschicht 42 könnte folglich zu einem erhöhten Reflux führen. Deshalb wird das SAP-Material 47 mit relativ großem Teilchendurchmesser verwendet.

Verbleibende Flüssigkeitsmengen, die nicht von der ersten Absorptionsschicht 42 absorbiert werden, werden zu der angrenzenden Zwischenschicht 43 weitergeleitet. Die Zwischenschicht 43 dient dazu, die erste und die zweite Absorptionsschicht 42, 44 voneinander zu trennen und die von der ersten Absorptionsschicht 42 nicht absorbierte Flüssigkeit breitflächig zu verteilen und an die zweite Absorptionsschicht 44 weiterzuleiten. Hierzu ist die Zwischenschicht 43 elastisch, aus einem Vliesmaterial 48 aus Baumwolle oder einem synthetischen baumwollartigen Material ausgebildet. Unter synthetischem baumwollartigen Material wird hier bspw. ein cotonisiertes Material oder ein aus Bikomponentenfasern bestehendes Material verstanden, das im Wesentlichen gleiche Eigenschaften wie ein aus natürlicher Baumwollfaser bestehendes Material aufweist. Insbesondere entsprechen eine Faserdicke und Faserlänge des synthetischen baumwollartigen Materials im Wesentlichen denjenigen eines natürlichen Baumwollmaterials. Geeignete Materialien auf Bikomponentenfaserbasis können z.B. aus Polyethylen/Polypropylen bestehen und sind z.B. unter dem Handelsnamen AL-Delta von ES FiberVisions, USA, kommerziell erhältlich.

Unabhängig davon, ob das Material 48 der Zwischenschicht 43 aus natürlicher oder synthetischer "Baumwolle" besteht, sind die Faserdicke, Faserlänge und die Vorzugsorientierung der Fasern derart gewählt, dass eine Flüssigkeitsverteilung der aufgenommenen Flüssigkeit in der Ebenenrichtung 33, also in der Fläche gefördert wird. Der Flüssigkeitstransport in der Ebenenrichtung 33 ist höher oder stärker als derjenige in Dickenrichtung 34. Hierzu sind eine hohe Faserlänge und hohe Sperrigkeit des Materials ebenso von Vorteil wie eine Faservorzugsausrichtung im Wesentlich in der Ebenenrichtung 33.

In bevorzugten Ausführungsformen weist die Zwischenschicht ein Flächengewicht von etwa 40 g/qm und eine Dicke von etwa 0,8 mm auf. Im Allgemeinen dürfte eine Dicke von etwa 0,7-0,9 mm geeignet sein, um die erste und zweite Absorptionsschicht 42, 44 wirksam voneinander zu trennen und die gewünschte breitflächige Flüssigkeitsverteilung zu erzielen, ohne die Dicke des Kerns 11 unnötig zu vergrößern.

Im Übrigen haben die beschriebenen Vliesmaterialien 48 der Zwischenschicht 43 gegenüber auf Zellulose basierenden Materialien, die alternativ auch eingesetzt werden könnten, eine um das Mehrfache bessere Flüssigkeitsverteilung in der Fläche. Deshalb wird auch in der Zwischenschicht 43 vorzugsweise keine Zellulose verwendet. Somit kann ein Absorptionskern 11 geschaffen werden, der vollkommen frei von Zellulose ist, was auch ein reduziertes Gewicht und Volumen des Kerns 11 zur Folge hat.

Der Mittel- bzw. Zwischenschicht 43 kommt eine zentrale Bedeutung zu, weil durch die breite Verteilung der Flüssigkeit in der Fläche das SAP-Material 47 der nächsten Schicht, also der zweiten Absorptionsschicht 44, im Einsatz in größerem Umfang und gleichmäßiger angewandt wird.

Auf die Zwischenschicht 43 folgt in Dickenrichtung zu der körperfernen Seite hin die zweite Absorptionsschicht 44, die wiederum ein Absorptionsmaterial 49 aufweist, das vorzugsweise aus reinem teilchenförmigen SAP-Material besteht. Das SAP-Material 49 bildet also im Wesentlichen 100% des gesamten Absorptionsmaterials der zweiten Absorptionsschicht 44. Jedenfalls ist das Absorptionsmaterial 49 der zweiten Absorptionsschicht 44 vorzugsweise frei von Zellulose. Die bereits oben in Verbindung mit dem SAP-Material 47 der ersten Absorptionsschicht 42 erwähnten Vorteile der hohen, gleichmäßigen Absorption, des reduzierten Gewichts und Volumens und der hohen Trocknungsgeschwindigkeit der Oberflächen treffen auch für das SAP-Material 49 der zweiten Absorptionsschicht 44 zu.

Das SAP-Material 49 der zweiten Absorptionsschicht 44 weist jedoch im Vergleich zu dem SAP-Material 47 der ersten Absorptionsschicht 42 einen kleineren Teilchen- bzw. Kerndurchmesser auf. Bspw. weist das SAP-Material 49 einen mittleren Teilchen- bzw. Kerndurchmesser von etwa 60-80 Mesh auf, was etwa 0,250 bis 0,177 mm entspricht. Wiederum kann der mittlere Teilchendurchmesser des SAP-Materials 49 je nach Zusammensetzung des SAP-Materials 49 geringfügig nach oben oder unten variieren. Der kleine Teilchendurchmesser ist speziell gewählt, um die restliche Flüssigkeitsmenge, die die erste Absorptionsschicht 42 und die Zwischenschicht 43 passiert, wirksam aufzunehmen und gut und langfristig zu halten. Die kleineren Partikel des SAP-Materials 49 können aufgrund ihrer größeren Oberfläche im Verhältnis zum Gewicht in der Summe mehr Flüssigkeit aufnehmen als die größeren Partikel des SAP-Materials 47. Außerdem können die kleinen Polymerteilchen des SAP-Materials 49 die Flüssigkeit besser dauerhaft einschlie-ßen. Ferner sind sie auch gegen Druck unempfindlicher, was den Reflux verhindert oder zumindest deutlich reduziert. Aus diesen Gründen sind die relative Anordnung der Absorptionsschichten 42, 44 und deren SAP-Teilchengrößen für die hohe Wirksamkeit des Absorptionskerns 11 von großer Bedeutung.

In einer bevorzugten Ausführungsform werden für eine Windel 1 der Größe M/3 für ein Körpergewicht des Trägers von 6-11 kg etwa 6-7 g SAP-Material 49 für die zweite Absorptionsschicht 44 verwendet. Die 0,9% NaCl-Absorption des SAP-Materials 49 sollte wenigstens 60 ml, vorzugsweise wenigstens 65 ml pro Gramm SAP betragen. Die Frischwasserabsorption sollte nicht weniger als 120 ml pro Gramm SAP betragen. Vorzugsweise beträgt sie wenigstens 130 ml. In einer vorteilhaften Ausführungsform wird ein SAP-Material 49 mit einer Frischwasserabsorption von 130-150 ml pro Gramm SAP verwendet. Derartige Leistungsmerkmale können derzeit bspw. mit SAP-Materialien von Sumitomo Seika Chemicals Co., Ltd., Japan, erhalten werden.

Die Unterschicht 46, die die letzte Schicht der Schichtstruktur des Absorptionskerns 11 bildet, besteht wieder aus einem Vlies (Non-Woven-Material) wie die Oberschicht 41. Im Unterschied zu der Oberschicht 41 kann das Vlies der Unterschicht 46 aber vorzugsweise hydrophob, also wasserabweisend sein, um dabei zu unterstützen, die absorbierte Flüssigkeit am Hindurchtreten zu den äußeren Lagen 31, 32 des Grundkörpers 9 zu hindern. Hierzu ist auch eine kurzfaserige Vliesvariante für die Unterschicht 46 von Vorteil, während das Vlies der Oberschicht 41 wegen ihrer Funktion eher langfaserig gewählt werden sollte. Das Vlies der Unterschicht 46 sollte besonders reißfest sein, um den Absorptionskern 11 stabil zu halten. Ein Material der Stärke von 11-13 g/qm hat sich in Ausführungsformen der Erfindung als vorteilhaft erwiesen.

Wie insbesondere aus Fig. 2 ersichtlich, kann die ADL-Schicht 29 in der dargestellten bevorzugten Ausführungsform an den Seitenrändern 36-39 des Absorptionskerns 11, an den Schichten 41-43 vorbei, bis zu oder nahe der Unterschicht 46 verlängert sein, um auch in diesen Bereichen ihre Aufnahme- und Verteilungsfunktion zu erfüllen und die Schichten 41-44 des Kerns 11 zu umgreifen und zu halten. Zusätzlich oder alternativ, kann die Unterschicht 46 mit der Oberschicht 41 an den Seitenrändern 36-39 des Absorptionskerns 11 mit einem toxisch, umweltmäßig und ökologisch unbedenklichen Klebstoff teilflächig verklebt sein, um dadurch den Kern 11 noch fester in der Windel 1 zu halten und sein Verrutschen zu verhindern. Eine Fixierung der Absorptionsschichten 42, 44 an der Oberschicht 41 oder der Unterschicht 46 des Kerns 11 kann entfallen. Durch die in der Ebenenrichtung 33 kontinuierliche Ausbildung der Absorptionsschichten 42, 44 weist die Schichtstruktur des Absorptionskerns 11 eine genügende Eigenstabilität auf, damit die Absorptionsschichten 42, 44 festgehalten und nicht verrutschen können und auch der Absorptionskern 11 insgesamt in dem Grundkörper 9 fest und unverrutschbar gehalten ist. Die Menge an erforderlichem Klebstoff kann insbesondere gegenüber herkömmlichen Windeln mit klassischem SAP/Pulp-Absorptionskern deutlich reduziert werden. Es können Kosten für den Klebstoff eingespart und Gewicht und Volumen bzw. Größe sowohl des Absorptionskerns 11 als auch der Windel 1 insgesamt reduziert werden.

Nachstehende Tabelle 1 listet das Gewicht des reinen SAP-Absorptionskerns 11 und der resultierenden Windel 1 für unterschiedliche Windeltypen und -größen gemäß bevorzugten Ausführungsformen der Erfindung auf. Das Gewicht des Absorptionskerns kann gegenüber vergleichbaren herkömmlichen Absorptionskernen auf Pulp/SAP-Basis um wenigstens 30-40% reduziert werden.

**Tabelle 1**

| **Typ** | **(Form) Größe** | **Trägergewicht** | **Größe** | **Windel gewicht** | **Kerngewicht** |
|---|---|---|---|---|---|
| Windel | NB | 0-5kg | 1 | 18 | 9,9 |
| Windel | S | 4-8kg | 2 | 22 | 11,1 |
| Windel | M | 6-11kg | 3 | 27 | 15,5 |
| Windel | L | 9-14kg | 4 | 30 | 17,2 |
| Windel | XL | 12-20kg | 5 | 33 | 18,9 |
| Windel | XXL | 16-28kg | 6 | 34 | 19,6 |
| Windelhöschen | Pull-up M | 6-11kg | 3 | 31 | 17,6 |
| Windelhöschen | Pull-up L | 9-14kg | 4 | 32 | 18,4 |
| Windelhöschen | Pull-up XL | 12-20kg | 5 | 35 | 19,9 |
| Windelhöschen | Pull-up XXL | 16-28kg | 6 | 36 | 20,5 |

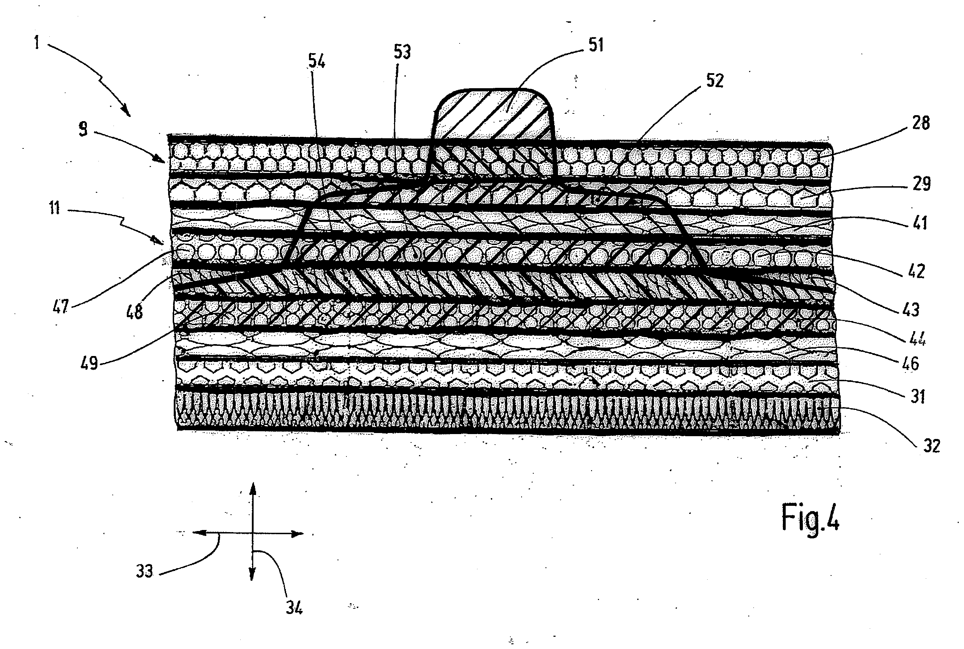

Zur Erläuterung der Funktionsweise der Windel 1 wird angenommen, dass diese bereits um die Taille eines Trägers, bspw. Babys, herum gelegt und mittels des Befestigungssystems 21 befestigt worden ist, so dass die Bundabschnitte 12, 13 die Taille umschließen und die Bündchen 24, 26 die Beinabschnitte des Trägers dichtend umfassen. Die Windel 1 dient dazu, Urin, Kot und andere aus dem Körper des Trägers ausgeschiedene Flüssigkeiten aufzunehmen und zu halten. Die Funktionsweise der Windel 1 mit dem erfindungsgemäßen Absorptionskern 11 soll nachstehend zusätzlich in Verbindung mit Fig. 4 näher erläutert werden.

In Fig. 4 ist der Schichtaufbau der Windel 1 in einer ausschnittsweisen und vergrößerten Darstellung gesondert veranschaulicht. Es ist ferner eine Flüssigkeitssäule 51 schematisch mit dargestellt, die die ausgeschiedene Körperflüssigkeit darstellen soll, wie sie auf die obere Lage 28 des Grundkörpers 9 der Windel 1 auftrifft. Die obere Lage 28 leitet diese Flüssigkeitssäule 51 schnell zu der darunter liegenden Aufnahme- und Verteilungs(ADL)-Schicht 29 ab. Diese verteilt die Flüssigkeit wirksam in der Ebenenrichtung 33, also in der Fläche, um eine möglichst große Oberfläche des Absorptionskerns 11 zu benetzen. Der in die Fläche verteilte Flüssigkeitsteil in der ADL-Schicht 29 ist in Fig. 4 durch das Bezugszeichen 52 angezeigt.

Die Oberschicht 41 des Absorptionskerns 11 transportiert die ankommende Flüssigkeit schnell zu der darunter liegenden ersten Absorptionsschicht 42 weiter. Das SAP-Material 47 der ersten Absorptionsschicht 42 absorbiert die Flüssigkeit und speichert diese. Aufgrund des großen Teilchendurchmessers des SAP-Materials 47 in der ersten Absorptionsschicht 42 kann die Absorption der Flüssigkeit mit hoher Geschwindigkeit und in großen Mengen bewerkstelligt werden, ohne dass das SAP-Material 49 an seine Kapazitätsgrenzen gelangt. Durch eine geringfügige Diffusion der Flüssigkeit in der Ebenenrichtung 33 wird die Flüssigkeit auch in der Fläche des kontinuierlichen SAP-Materials 47 verteilt, wie dies durch den sich geringfügig erweiternden Teil 53 der Flüssigkeit in der ersten Absorptionsschicht 42 angezeigt ist.

Der von dem SAP-Material 47 der ersten Absorptionsschicht 42 nicht aufgenommene Teil gelangt zu der Zwischenschicht 43, die die Flüssigkeit sehr effektiv breitflächig verteilt und an die zweite Absorptionsschicht 44 weiterleitet. Die breite Flüssigkeitsverteilung in der Zwischenschicht 43 ist in Fig. 4 schematisch dargestellt und durch das Bezugszeichen 54 angezeigt. Somit bewirkt die Zwischenschicht, dass eine sehr große Fläche der darunter liegenden zweiten Absorptionsschicht 44 von der Flüssigkeit benetzt wird und somit ein Großteil des SAP-Materials 49 in dieser Schicht zur Absorption genutzt werden kann.

Das SAP-Material 49 der zweiten Absorptionsschicht 44 absorbiert die Flüssigkeit und hält diese dauerhaft und sicher zurück. Die SAP-Polymerteilchen mit kleinem Teilchendurchmesser schließen die Flüssigkeit dauerhaft ein. Sie verhindern auch auf effiziente Weise, dass ein von außen auf die Windel 1 ausgeübter Druck oder eine Bewegung der Windel 1 einen Rückfluss der Flüssigkeit in der Dickenrichtung 34 nach oben zu der oberen Lage 28 der Windel 1 und aus dieser heraus zu der Haut des Trägers bewirkt. Vielmehr kann die absorbierte Flüssigkeit sicher und langfristig in den beiden Absorptionsschichten 42, 44 des Absorptionskerns 11 gespeichert werden.

Die erfindungsgemäße Windel 1 zeichnet sich durch ein besonders gutes Absorptionsvermögen mit hoher Absorptionsgeschwindigkeit und hoher Absorptionskapazität auf, was auf die geeignete Auswahl der Materialien und Eigenschaften für die erste und zweite Absorptionsschicht 42, 44 und die Zwischenschicht 43, eine geeignete Abstimmung derselben aufeinander, einschließlich der passenden, unterschiedlichen Teilchendurchmesser der Absorptionsschichten 42, 44, und die Flüssigkeitsverteilungsfunktion der Zwischenschicht 43 zurückzuführen ist. Die Saugfähigkeit, Absorptionsmenge, Absorptionszeit und Verteilung der Flüssigkeit in dem Absorptionskern 11 können deutlich verbessert werden, und der Reflux kann minimiert werden. Das Gewicht und die Dicke des Kerns können gegenüber herkömmlichen SAP/Pulp-Absorptionskernen deutlich reduziert werden. Dies hat auch eine Reduktion des Gewichts und der Größe der Windel 1 insgesamt zur Folge. Damit kann der Aufwand und können Kosten für Lagerung, Transport und Handhabung, z.B. auch für die Verkaufsfläche, gesenkt werden.

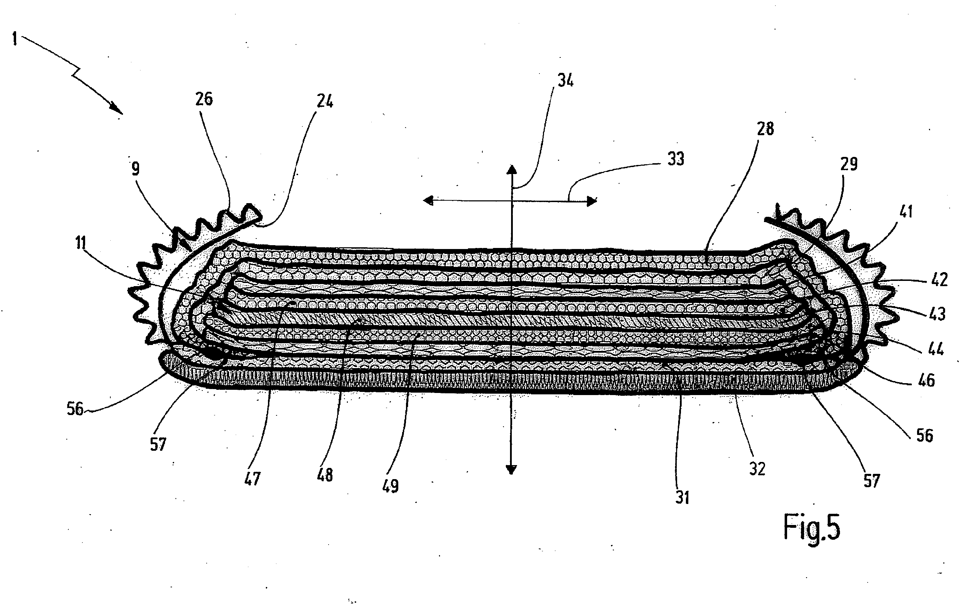

Fig. 5 zeigt eine modifizierte Ausführungsform einer Windel bzw. eines Windelhöschens 1 gemäß der Erfindung. Soweit Übereinstimmung im Aufbau und/oder in der Funktion mit der vorstehend beschriebenen Ausführungsform gemäß den Figuren 1-4 besteht, wird unter Zugrundelegung gleicher Bezugszeichen auf die vorstehende Beschreibung Bezug genommen.

Die Ausführungsform nach Fig. 5 unterscheidet sich von derjenigen nach den Figuren 1-4 im Wesentlichen dadurch, dass hier die obere Lage 28 nicht in der Nähe der Auslaufschutzschicht 31 des Grundkörpers 9 endet und dort mit dieser verklebt ist. Vielmehr ist die die obere Lage 28 hier noch weiter verlängert und auf Höhe der Auslaufschutzschicht 31 derart umgeschlagen, dass sie mit ihren Randabschnitten 56 den Absorptionskern 11 auch von dessen Unterseite geringfügig umgreift. Die Randabschnitte 56 der oberen Lage 28 erstrecken sich also zwischen der Unterschicht 46 des Kerns und der Auslaufschutzschicht 31 vorzugsweise über etwa 10-20 mm in Seitenrichtung aufeinander zu und sind dort an einer Stelle 57 mit der Auslaufschutzschicht 31 des Grundkörpers 9 schmalflächig verklebt. Vorteilhafterweise kann dadurch eine Ausscheidungsflüssigkeit, wie bspw. Urin, die zur Seite (rechts und links in Fig. 5) aus dem Absorptionskern 11 austritt, auch unten, im Bereich der Seitenränder des Auslaufschutzschicht 31 absorbiert werden. Außerdem wird der Halt des Absorptionskerns 11 in dem Grundkörper noch weiter verbessert.

Im Rahmen der Erfindung sind noch weitere, vielfältige Modifikationen möglich. Während die vorstehende Beschreibung auf eine Windel oder ein Windelhöschen 1 Bezug nimmt, ist die Erfindung, wie vorstehend erläutert, auch auf andere Einweg-Absortptionsartikel und -Hygieneartikel anwendbar. Die Windel kann zusätzlich unterschiedliche auf dem vorliegenden Gebiet bekannte Merkmale, wie Flügelfelder, Taillenverschlusselemente, Gummibänder und dgl. aufweisen, um eine bessere Sitz- bzw. Passform zu erhalten, eine bessere Dichtigkeit und Rückhaltung für die Flüssigkeit zu erzielen und/oder die Ästhetik zu verbessern. Bspw. kann bei einer Anwendung für Windelhöschen außer den in den Figuren 1-5 gezeigten Ausführungsformen, die im Unterschied zu einer Einwegwindel 1 mit durchgängigem elastischen Bündchen 16, 17 versehen sind, in einer weiteren, bevorzugten (nicht gezeigten) Ausführungsform eine Grundform vorteilhaft sein, die auf beiden Seiten um die Längsachse 2, entlang und parallel zu dieser zusätzlich vernäht ist, ähnlich wie in Fig. 1 mit den elastischen Fäden 27 angezeigt. Dadurch kann eine spezielle Verstärkung geschaffen werden, die den Auslaufschutz des Windelhöschen weiter verbessert, da sie für ein besser Anliegen dessen unten Bereiches sorgt.

In einer weiteren (nicht gezeigten) Azsführungsform kann auch ein Nässeindikator vorgesehen sein, der an der Außenseite des Absorptionsartikels angebracht und von außen gut sichtbar ist, um einen Nässezustand im Inneren des Absorptionsartikels und gegebenenfalls den Nässegrad anzuzeigen. Der Nässeindikator befindet sich vorzugsweise hinter der unteren Außenvliesschicht 46 und kann die Kleidung oder Bettwäsche nicht verschmutzen.

Es können auch alternative Materialien für den Absorptionskern 11 und die Windel 1 insgesamt verwendet werden, die aus nachwachsenden Rohstoffen bestehen, um eine weitgehend oder vollständig kompostierbare Windel zu schaffen. Bspw. können die synthetischen Non-Woven-Materialschichten 28, 41, 43, 46 und 47 durch natürliche Stoffe, wie etwa Bambusfaserstoffe oder durch speziell behandelte Zellulose ersetzt werden. Es ist möglich, Superabsorber aus Stärke und anderen heutzutage bekannten oder künftig entwickelten natürlichen Superabsorbermaterialien herzustellen. Die Trenn- bzw. Zwischenschicht 43 kann auch aus Naturbaumwolle bestehen. Aufgrund der deutlich verbesserten Absorption, der besseren Steuerbarkeit der Flüssigkeitsverteilung und der reduzierten Kosten werden die synthetischen Materialien, insbesondere die hierin angegebenen, vorgezogen.

Es ist ein Einweg-Absorptionsartikel 1, wie etwa eine Einweg-Windel oder ein Einweg-Windelhöschen, mit einem Grundkörper 9 und einem in dem Grundkörper 9 aufgenommenen Absorptionskern 11 offenbart. Der Absorptionskern 11 weist wenigstens eine erste Absorptionsschicht 42 und eine zweite Absorptionsschicht 44 zur Absorption einer durch den Absorptionsartikel 1 aufgenommenen Flüssigkeit auf. Die erste und die zweite Absorptionsschicht 42, 44 liegen in Dickenrichtung 34 übereinander und sind durch eine Zwischenschicht 43 voneinander getrennt, die eingerichtet ist, um Flüssigkeit von der ersten zu der zweiten Absorptionsschicht 42, 44 zu leiten und auf die zweite Absorptionsschicht 44 flächig zu verteilen. Die erste Absorptionsschicht 42 weist ein teilchenförmiges, superabsorbierendes Polymermaterial 47 mit einem ersten Teilchendurchmesser auf, und die zweite Absorptionsschicht 44 weist ein teilchenförmiges, superabsorbierendes Polymermaterial 49 mit einem zweiten Teilchendurchmesser auf. Der zweite Teilchendurchmesser ist kleiner als der erste Teilchendurchmesser.

### Bezugszeichenliste:

- 1: Windel, Einweg-Absorptionsartikel
- 2: Längsachse
- 3: Querachse
- 4: oberer Rand
- 6: unterer Rand
- 7: linker Rand
- 8: rechter Rand
- 9: Grundkörper
- 11: Absorptionskern
- 12: hinterer Bundabschnitt
- 13: vorderer Bundabschnitt
- 14: Zwischenabschnitt
- 16: elastisches Bündchenelement
- 17: Schlaufen- oder Flauschband
- 18: hintere Randabschnitte
- 19: vorderer Randabschnitte
- 21: Befestigungssystem
- 22: Befestigungselemente
- 23: Anlegebereich
- 24: inneres Bündchen
- 26: äußeres Bündchen
- 27: elastische Elemente/Fäden
- 28: obere Lage
- 29, 29': Aufnahme- und Verteilungsschicht, ADL-Schicht
- 30: Klebestelle
- 31: Auslaufschutzschicht, PE-Folie
- 32: untere Lage
- 33: Ebenenrichtung
- 34: Tiefenrichtung
- 36: oberer Rand
- 37: unterer Rand
- 38: linker Seitenrand
- 39: rechte Seitenrand
- 41: Oberschicht
- 42: erste Absorptionsschicht
- 43: Zwischenschicht, Trennschicht
- 44: zweite Absorptionsschicht
- 46: Unterschicht
- 47: SAP-Material, Absorptionsmaterial
- 48: (Vlies)Material
- 49: SAP-Material, Absorptionsmaterial
- 51: Flüssigkeitssäule
- 52: Flüssigkeitsteil
- 53: Flüssigkeitsteil
- 54: Flüssigkeitsverteilung
- 56: Randabschnitte
- 57: Klebestelle

## Patentansprüche

1. Einweg-Absorptionsartikel, insbesondere Einweg-Windel oder Einweg-Windelhöschen, der aufweist:
einen Grundkörper (9), der eine im Wesentlichen flüssigkeitsdurchlässige obere Lage (28), die im Einsatz mit der Körperhaut eines Trägers in Kontakt gelangt, und eine unterer Lage (32) auf einer gegenüberliegenden, körperfernen Seite aufweist;
einen Absorptionskern (11), der zwischen der oberen Lage (28) und der unteren Lage (32) des Grundkörpers (9) angeordnet ist und eine erste Absorptionsschicht (42) und eine zweite Absorptionsschicht (44) zur Absorption einer durch den Absorptionsartikel (1) aufgenommenen Flüssigkeit aufweist, wobei die erste und die zweite Absorptionsschicht (42, 44) in der Dickenrichtung (34) zwischen der oberen und der unteren Lage (28, 32) übereinanderliegen;
wobei die erste Absorptionsschicht (42) ein teilchenförmiges superabsorbierendes Polymermaterial (47) mit einem ersten Teilchendurchmesser aufweist und die zweite Absorptionsschicht (44) ein teilchenförmiges superabsorbierendes Polymermaterial (49) mit einem zweiten Teilchendurchmesser aufweist, der kleiner ist als der erste Teilchendurchmesser, und
wobei zwischen der ersten und der zweiten Absorptionsschicht (42, 44) eine Zwischenschicht (43) eingefügt ist, die die erste und zweite Absorptionsschicht (42, 44) voneinander trennt und eingerichtet ist, um Flüssigkeit von der ersten zu der zweiten Absorptionsschicht (42, 44) zu leiten und auf die zweite Absorptionsschicht (44) flächig zu verteilen, wobei die Zwischenschicht (43) aus einem Vliesmaterial (48) aus Baumwolle oder einem synthetischen baumwollartigen Material ausgebildet ist, wobei die Faserdicke, Faserlänge und die Vorzugsorientierung der Fasern des Vliesmaterials (48) derart gewählt sind, dass eine Flüssigkeitsverteilung der aufgenommenen Flüssigkeit in einer Ebenenrichtung (33), in der Fläche gefördert wird und der Flüssigkeitstransport in der Ebenenrichtung (33) höher oder stärker als derjenige in der Dickenrichtung (34) ist.

2. Einweg-Absorptionsartikel nach Anspruch 1, wobei die erste Absorptionsschicht (42) an der körpernahen Seite, näher an der oberen Lage (28) des Absorptionsartikels (1) angeordnet ist und die zweite Absorptionsschicht (44) an der körperfernen Seite, näher an der unteren Lage (32) des Absorptionsartikels (1) angeordnet ist.

3. Einweg-Absorptionsartikel nach Anspruch 1 oder 2, wobei der Teilchendurchmesser des superabsorbierenden Polymermaterials (49) in der zweiten Absorptionsschicht (44) weniger als 70 %, vorzugsweise weniger als 60 %, am meisten bevorzugt weniger als 50 % des Teilchendurchmessers des superabsorbierenden Polymermaterials (47) in der ersten Absorptionsschicht (42) beträgt.

4. Einweg-Absorptionsartikel nach einem der vorhergehenden Ansprüche, wobei das superabsorbierende Polymermaterial (47) der ersten Absorptionsschicht (42) einen Teilchendurchmesser in einem Bereich von etwa 0,595-0,400 mm aufweist und das superabsorbierende Polymermaterial (49) der zweiten Absorptionsschicht (44) einen Teilchendurchmesser in einem Bereich von etwa 0,250-0,177 mm aufweist.

5. Einweg-Absorptionsartikel nach einem der vorhergehenden Ansprüche, wobei das superabsorbierende Polymermaterial (47) der ersten Absorptionsschicht (42) eine 0,9% NaCl - Absorption von wenigstens 40 ml, vorzugsweise wenigstens 50 ml pro Gramm superabsorbierendes Polymermaterial und eine Frischwasserabsorption von wenigstens 80 ml, vorzugsweise wenigstens 100 ml pro Gramm superabsorbierendes Polymermaterial aufweist und das superabsorbierende Polymermaterial (49) der zweiten Absorptionsschicht (44) eine 0,9% NaCl- Absorption von wenigstens 60 ml, vorzugsweise wenigstens 65 ml pro Gramm superabsorbierendes Polymermaterial und eine Frischwasserabsorption von wenigstens 120 ml, vorzugsweise wenigstens 130 ml pro Gramm superabsorbierendes Polymermaterial aufweist.

6. Einweg-Absorptionsartikel nach einem der vorhergehenden Ansprüche, wobei die erste und zweite Absorptionsschicht (42, 44) frei von Zellulose sind.

7. Einweg-Absorptionsartikel nach Anspruch 6, wobei das superabsorbierende Polymermaterial (47, 49) einziges Absorptionsmaterial in der ersten und zweiten Absorptionsschicht (42, 44) ist.

8. Einweg-Absorptionsartikel nach einem der vorhergehenden Ansprüche, wobei der Absorptionskern (11) für eine Windel der Größe M/3 für ein Trägergewicht von 6-11 kg ein Kerngewicht von weniger als 20 g, vorzugsweise weniger als 17 g, und für eine Windel der Größe XXL/6 für ein Trägergewicht von 16-28 kg ein Kerngewicht von weniger als 25 g, vorzugsweise weniger als 21 g, aufweist.

9. Einweg-Absorptionsartikel nach einem der vorhergehenden Ansprüche, wobei das superabsorbierende Polymermaterial (47, 49) in der ersten und der zweiten Absorptionsschicht (42, 44) in Längsrichtung zwischen gegenüberliegenden Endrändern und in Seitenrichtung zwischen gegenüberliegenden Seitenrändern des Absorptionskerns (11) jeweils kontinuierlich verteilt ist.

10. Einweg-Absorptionsartikel nach einem der vorhergehenden Ansprüche, wobei der Absorptionskern (11) ferner eine hydrophile Oberschicht (41) und eine vorzugsweise hydrophobe Unterschicht (46) aufweist, die die erste bzw. zweite Absorptionsschicht (42, 44) in Dickenrichtung bedecken und aus einem Vliesstoff bestehen.

11. Einweg-Absorptionsartikel nach einem der vorhergehenden Ansprüche, wobei die Unterschicht (46) mit der Oberschicht (41) an den Seitenrändern des Absorptionskerns (11) verklebt ist.

12. Einweg-Absorptionsartikel nach einem der vorhergehenden Ansprüche, der ferner eine Aufnahme- und Verteilungsschicht (29) aufweist, die zwischen der oberen Lage (28) des Grundkörpers (9) und dem Absorptionskern (11) eingefügt und eingerichtet ist, um aufgenommene Flüssigkeit schnell breitflächig an den Absorptionskern (11) weiterzuleiten.

13. Einweg-Absorptionsartikel nach Anspruch 12, wobei die obere Lage (28) und die Aufnahme/Verteilungsschicht (29) mit dem Absorptionskern (11) nicht verklebt sind.

14. Einweg-Absorptionsartikel nach einem der vorhergehenden Ansprüche, wobei die obere Lage (28) den Absorptionskern (11) an wenigstens zwei Seiten umgreift.

15. Einweg-Absorptionsartikel nach einem der vorhergehenden Ansprüche, wobei die untere Lage (32) des Grundkörpers (9) durch eine Vliesaußenschicht gebildet ist und zwischen der Vliesaußenschicht (32) und dem Absorptionskern (11) eine Auslaufschutzschicht (31) in Form einer flüssigkeitsundurchlässigen Folie, vorzugsweise aus Polyethylen oder Polypropylen, angeordnet ist.

## Claims

1. A disposable absorbent article, in particular a disposable diaper or disposable diaper panty, comprising:
a base body (9) which has a substantially liquid-permeable upper layer (28) which, in use, comes into contact with the body skin of a wearer, and a lower layer (32) on an opposite side remote from the body;
an absorbent core (11) disposed between the upper layer (28) and the lower layer (32) of the base body (9) and having a first absorbent layer (42) and a second absorbent layer (44) for absorbing a liquid received by the absorbent article (1), wherein the first and the second absorbent layer (42, 44) are superposed in the thickness direction (34) between the upper and the lower layer (28, 32);
wherein the first absorbent layer (42) comprises a particulate superabsorbent polymer material (47) with a first particle diameter and the second absorbent layer (44) comprises a particulate superabsorbent polymer material (49) having a second particle diameter which is smaller than the first particle diameter, and
wherein an intermediate layer (43) is interposed between the first and the second absorbent layer (42, 44), the intermediate layer (43) separating the first and the second absorbent layer (42, 44) from each other and arranged to convey liquid from the first to the second absorbent layer (42, 44) and to distribute it over the surface of the second absorbent layer (44), wherein the intermediate layer (43) is formed of a non-woven material (48) of cotton or a synthetic cotton-like material, wherein the fiber thickness, fiber length and preferred orientation of the fibers of the non-woven material (48) are selected such that a liquid distribution of the absorbed liquid in a plane direction (33), in the surface is promoted and the liquid transport in the plane direction (33) is higher or stronger than that in the thickness direction (34).

2. The disposable absorbent article according to claim 1, wherein the first absorbent layer (42) is arranged on the side near the body, closer to the upper layer (28) of the absorbent article (1) and the second absorbent layer (44) is arranged on the side distant from the body, closer to the lower layer (32) of the absorbent article (1).

3. The disposable absorbent article according to claim 1 or 2, wherein the particle diameter of the superabsorbent polymer material (49) in the second absorbent layer (44) is less than 70%, preferably less than 60%, most preferably less than 50% of the particle diameter of the superabsorbent polymer material (47) in the first absorbent layer (42).

4. The disposable absorbent article of anyone of the preceding claims, wherein the superabsorbent polymer material (47) of the first absorbent layer (42) has a particle diameter in a range of about 0.595 - 0.400 mm and the superabsorbent polymer material (49) of the second absorbent layer (44) has a particle diameter in a range of about 0.250 - 0.177 mm.

5. The disposable absorbent article according to anyone of the preceding claims, wherein the superabsorbent polymer material (47) of the first absorbent layer (42) has a 0.9% NaCl absorbtion of at least 40 ml, preferably at least 50 ml per gram of superabsorbent polymer material and a fresh water absorption of at least 80 ml, preferably at least 100 ml per gram of superabsorbent polymer material, and the superabsorbent polymer material (49) of the second absorbent layer (44) has a 0.9% NaCl absorption of at least 60 ml, preferably at least 65 ml per gram of superabsorbent polymer material and a fresh water absorption of at least 120 ml, preferably at least 130 ml per gram of superabsorbent polymer material.

6. The disposable absorbent article according to anyone of the preceding claims, wherein the first and the second absorbent layer (42, 44) are free of cellulose.

7. The disposable absorbent article of claim 6, wherein the superabsorbent polymer material (47, 48) is the only absorbent material in the first and the second absorbent layer (42, 44).

8. The disposable absorbent article according to anyone of the preceding claims, wherein the absorbent core (11) has a core weight of less than 20 g, preferably less than 17 g, for a diaper of size M/3 for a wearer weight of 6-11 kg, and a core weight of less than 25 g, preferably less than 21 g, for a diaper of size XXL/6 for a wearer weight of 16-28 kg.

9. The disposable absorbent article according to anyone of the preceding claims, wherein the superabsorbent polymer material (47, 49) in the first and the second absorbent layer (42, 44) is respectively continuously distributed in the longitudinal direction between opposite end edges and in the lateral direction between opposite side edges of the absorbent core (11).

10. The disposable absorbent article according to anyone of the preceding claims, wherein the absorbent core (11) further comprises a hydrophilic top layer (41) and a preferably hydrophobic bottom layer (46) which cover the first and the second absorbent layer (42, 44), respectively, in the thickness direction and are made of non-woven fabric.

11. The disposable absorbent article according to anyone of the preceding claims, wherein the bottom layer (46) is glued to the top layer (41) at the side edges of the absorbent core (11).

12. The disposable absorbent article according to anyone of the preceding claims, further comprising an acquisition and distribution layer (29) interposed between the upper layer (28) of the base body (9) and the absorbent core (11) and arranged to rapidly transfer received liquid over a wide area to the absorbent core (11).

13. The disposable absorbent article according to claim 12, wherein the upper layer (28) and the acquisition / distribution layer (29) are not glued to the absorbent core (11) .

14. The disposable absorbent article according to anyone of the preceding claims, wherein the upper layer (28) embraces the absorbent core (11) on at least two sides.

15. The disposable absorbent article according to anyone of the preceding claims, wherein the lower layer (32) of the base body (9) is formed by a non-woven outer layer and a leakage protection layer (31) in the form of a liquid-impermeable film, preferably made of polyethylene or polypropylene, is arranged between the non-woven outer layer (32) and the absorbent core (11).

## Revendications

1. Article d'absorption jetable, en particulier couche jetable ou couche-culotte jetable, qui comprend :
un corps de base (9) qui présente une feuille supérieure (28), sensiblement perméable aux liquides, qui, lors de l'utilisation, entre en contact avec la peau du corps d'un utilisateur, et une feuille inférieure (32) située sur un côté opposé, éloigné du corps ;
un noyau d'absorption (11) qui est disposé entre la feuille supérieure (28) et la feuille inférieure (32) du corps de base (9) et présente une première couche d'absorption (42) et une deuxième couche d'absorption (44) destinées à absorber un liquide recueilli par l'article d'absorption (1), la première et la deuxième couche d'absorption (42, 44) étant superposées dans le sens de l'épaisseur (34) entre les feuilles supérieure et inférieure (28, 32) ;
la première couche d'absorption (42) présentant un matériau polymère super-absorbant (47) sous forme de particules, avec un premier diamètre de particules, et la deuxième couche d'absorption (44) présentant un matériau polymère super-absorbant (49) sous forme de particules, avec un deuxième diamètre de particules qui est plus petit que le premier diamètre de particules, et
une couche intermédiaire (43) étant intercalée entre la première et la deuxième couche d'absorption (42, 44), qui sépare l'une de l'autre les première et deuxième couches d'absorption (42, 44) et est conçue pour guider un liquide de la première à la deuxième couche d'absorption (42, 44) et pour le répartir en surface sur la deuxième couche d'absorption (44), sachant que la couche intermédiaire (43) est constituée d'un matériau non tissé (48) en coton ou matériau synthétique de type coton, l'épaisseur de fibre, la longueur des fibres et l'orientation préférentielle des fibres du matériau non tissé (48) étant choisies de manière à favoriser une répartition du liquide absorbé dans le sens d'un plan (33), dans la surface, et de manière à ce que le transport du liquide soit plus élevé ou plus important dans le sens du plan (33) que dans le sens de l'épaisseur (34).

2. Article d'absorption jetable selon la revendication 1, dans lequel la première couche d'absorption (42), située près du corps, est disposée plus près de la feuille supérieure (28) de l'article d'absorption (1), et la deuxième couche d'absorption (44), éloignée du corps, est disposée plus près de la feuille inférieure (32) de l'article d'absorption (1).

3. Article d'absorption jetable selon la revendication 1 ou 2, dans lequel le diamètre des particules du matériau polymère super-absorbant (49) dans la deuxième couche d'absorption (44) est inférieur à 70 %, de préférence inférieur à 60 %, et de manière particulièrement avantageuse inférieur à 50 % du diamètre des particules du matériau polymère super-absorbant (47) de la première couche d'absorption (42).

4. Article d'absorption jetable selon l'une des revendications précédentes, dans lequel le matériau polymère super-absorbant (47) de la première couche d'absorption (42) présente un diamètre de particules compris dans une plage allant d'environ 0,595 à 0,400 mm, et le matériau polymère super-absorbant (49) de la deuxième couche d'absorption (44) présente un diamètre de particules compris dans une plage allant de 0,250 à 0,177 mm.

5. Article d'absorption jetable selon l'une des revendications précédentes, dans lequel le matériau polymère super-absorbant (47) de la première couche d'absorption (42) présente une absorption de NaCl 0,9 % d'au moins 40 ml, de préférence d'au moins 50 ml par gramme de matériau polymère super-absorbant, et une absorption d'eau fraîche d'au moins 80 ml, de préférence d'au moins 100 ml par gramme de matériau polymère super-absorbant, et le matériau polymère super-absorbant (49) de la deuxième couche d'absorption (44) présente une absorption de NaCl 0,9 % d'au moins 60 ml, de préférence d'au moins 65 ml par gramme de matériau polymère super-absorbant, et une absorption d'eau fraîche d'au moins 120 ml, de préférence d'au moins 130 ml par gramme de matériau polymère super-absorbant,

6. Article d'absorption jetable selon l'une des revendications précédentes, dans lequel les première et deuxième couches d'absorption (42, 44) sont exemptes de cellulose.

7. Article d'absorption jetable selon la revendication 6, dans lequel le matériau polymère super-absorbant (47, 49) est l'unique matériau d'absorption dans les première et deuxième couches d'absorption (42, 44).

8. Article d'absorption jetable selon l'une des revendications précédentes, dans lequel le noyau d'absorption (11), pour une couche de la taille M/3 pour un poids de l'utilisateur allant de 6 à 11 kg, présente un poids de noyau inférieur à 20 g, de préférence inférieur à 17 g, et pour une couche de la taille XXL/6 pour un poids de l'utilisateur allant de 16 à 28 kg, un poids de noyau inférieur à 25 g, de préférence inférieur à 21 g.

9. Article d'absorption jetable selon l'une des revendications précédentes, dans lequel le matériau polymère super-absorbant (47, 49) dans la première et la deuxième couche d'absorption (42, 44) est réparti de façon continue, respectivement dans le sens longitudinal entre des bords d'extrémité opposés et dans le sens latéral entre des bords latéraux opposés du noyau d'absorption (11).

10. Article d'absorption jetable selon l'une des revendications précédentes, dans lequel le noyau d'absorption (11) présente en outre une couche supérieure (41) hydrophile et une couche inférieure (46), de préférence hydrophile, qui recouvrent respectivement la première et la deuxième couche d'absorption (42, 44), dans le sens de l'épaisseur, et sont constituées d'un matériau non tissé.

11. Article d'absorption jetable selon l'une des revendications précédentes, dans lequel la feuille inférieure (46) est collée à la feuille supérieure (41) sur les bords latéraux du noyau d'absorption (11).

12. Article d'absorption jetable selon l'une des revendications précédentes, qui présente en outre une couche d'absorption et de répartition (29) qui est intercalée entre la feuille supérieure (28) du corps de base (9) et le noyau d'absorption (11) et est conçue pour transmettre rapidement le liquide absorbé sur une grande surface du noyau d'absorption (11).

13. Article d'absorption jetable selon la revendication 12, dans lequel la feuille supérieure (28) et la couche d'absorption/de répartition (29) ne sont pas collées au noyau d'absorption (11).

14. Article d'absorption jetable selon l'une des revendications précédentes, dans lequel la feuille supérieure (28) entoure le noyau d'absorption (11) sur au moins deux côtés.

15. Article d'absorption jetable selon l'une des revendications précédentes, dans lequel la feuille inférieure (32) du corps de base (9) est constituée d'une feuille externe de non-tissé, et une feuille anti-fuite (31), réalisée sous la forme d'un film imperméable aux liquides, de préférence en polyéthylène ou en polypropylène, est disposée entre la feuille externe de non-tissé (32) et le noyau d'absorption (11).
